# EUROPEAN PATENT APPLICATION

(11) **EP 4 011 397 A1**
(43) Date of publication of application: **15.06.2022**
(21) Application number: 20849709.9
(22) Date of filing: 03.08.2020
(51) Int. Cl.: A61K 48/00, A61P 25/28, C07K 14/47, C12N 15/12, A61K 38/16, A61K 31/685, A61K 31/7088

(54) **AGENT FOR PREVENTING OR TREATING MILD COGNITIVE IMPAIRMENT**

(30) Priority: 07.08.2019 JP 2019145259
(71) Applicant: National University Corporation Tokyo Medical and Dental University, Tokyo 113-8510 (JP)
(72) Inventor: OKAZAWA, Hitoshi, Tokyo 113-8510 (JP)
(74) Representative: Hamer, Christopher K.
(86) International application number: PCT/JP2020/029677
(87) International publication number: WO 2021/024986

(57) **Abstract**

An object of the present invention is to provide a substance that can prevent or treat mild cognitive impairment. Mammalian YAP, a polynucleotide encoding mammalian YAP, or a substance capable of increasing an amount of mammalian YAP in a nucleus of a brain neuron is used in the prevention or treatment of mild cognitive impairment.

## Description

### Technical Field

The present invention relates to a formulation for preventing and/or treating mild cognitive impairment (hereinafter, also referred to as "MCI"), comprising mammalian YAP (Yes-associated protein), a polynucleotide encoding mammalian YAP, or a substance capable of increasing an amount of mammalian YAP in a nucleus of a brain neuron (hereinafter, these components are also referred to as the "present YAP-related substance").

### Background Art

Alzheimer's dementia (Alzheimer's disease) (hereinafter, also referred to as "AD") is an irreversible and progressive central nervous disease and is known to manifest symptoms such as cognitive dysfunction associated with memory loss and impaired thinking (dementia), behavior disorder, and personality change. Alzheimer's dementia is the most common disease among dementia patients and accounts for approximately 60 to 80% of the entire dementia patients. Alzheimer's dementia generally develops in elderly people at least 65 years old and sometimes develops at age 64 years or younger. In this case, it is called early-onset Alzheimer's disease.

According to the announcement of the Cabinet Office of Japan, the number of dementia patients in Japan in 2012 is 4,620,000 people, which mean one person in seven of elderly people at least 65 years old, and is expected to increase to approximately 7,000,000 people in 2025 (one person in five). Such an increased number of patients with dementia such as Alzheimer's dementia presumably aggravates economical or psychological burdens on the nation or relevant people of the patients due to escalation of medical costs, nursing care problems, etc.

MCI patients have decline in cognitive function, which however does not satisfy the diagnostic criteria of dementia, and find no problem in fundamental daily life or social life. Some MCI patients are considered to develop Alzheimer's dementia in the future. Therefore, if MCI can be treated, the prevention of Alzheimer's dementia which may be developed in the future can be expected.

Particular compositions (ferulic acid, ginkgo leaf extracts, and α-glycerophosphocholine) have been reported to have an effect of ameliorating the symptoms of MCI (patent document 1). Also, hydrogen has been reported to have a prophylactic effect on MCI (patent document 2).

### Prior Art Documents

### Patent Documents

Patent Document 1: Japanese unexamined Patent Application Publication No. 2019-6699
Patent Document 2: International Publication No. WO 2018/012596

### Summary of the Invention

### Object to be Solved by the Invention

An object of the present invention is to provide a substance that can prevent or treat mild cognitive impairment.

### Means to Solve the Object

The present inventor has pursued diligent studies to attain the object and consequently confirmed that: 1) the concentration of HMGB1 known as one of DAMPs (damageassociated molecular patterns) released from necrotic cells is higher in cerebrospinal fluid (CSF) samples derived from MCI patients than in CSF samples derived from individuals having no MCI; and 2) Hippo pathway-dependent necrosis is increased in the brain neurons of MCI patients. Meanwhile, sphingosine 1-phosphate (S1P), a substance promoting the nuclear translocation of YAP, as well as YAP and its isoforms are known to have an effect of suppressing Hippo pathway-dependent necrosis. Accordingly, the present inventor has analyzed whether to suppress Hippo pathway-dependent necrosis caused by MCI using S1P and a YAP isoform (specifically, rat YAPΔC-ins61), and consequently completed the present invention by finding that an amount of YAP decreased by MCI is increased in a nuclei of brain neurons and Hippo pathway-dependent necrosis in these brain neurons is suppressed; thus decline in cognitive function can be prevented or ameliorated.

Specifically, the present invention is as follows.
[1] An agent for preventing or treating mild cognitive impairment, comprising mammalian YAP, a polynucleotide encoding mammalian YAP, or a substance capable of increasing an amount of mammalian YAP in a nucleus of a brain neuron.
[2] The agent according to the above [1], wherein the mammalian YAP is a polypeptide comprising an amino acid sequence having at least 80% sequence identity to the amino acid sequence shown by SEQ ID NO: 1, and the substance capable of increasing an amount of mammalian YAP in a nucleus of a brain neuron is sphingosine 1-phosphate.
[3] The agent according to the above [1] or [2], wherein the mild cognitive impairment is a condition having an elevated concentration of HMGB1 in cerebrospinal fluid as compared with a control subject without mild cognitive impairment.
[4] The preventing or treating agent according to the above [3], wherein the control subject without mild cognitive impairment is a healthy individual or an Alzheimer's disease patient.

Other embodiments of the present invention can provide: a method for preventing or treating mild cognitive impairment, comprising the step of administering the present YAP-related substance to a subject in need of the prevention or treatment of mild cognitive impairment; the present YAP-related substance for use as an agent for preventing or treating mild cognitive impairment; the present YAP-related substance for use in the prevention or treatment of mild cognitive impairment; and use of the present YAP-related substance for producing an agent for preventing or treating mild cognitive impairment.

### Effect of the Invention

The present YAP-related substance has an effect of suppressing Hippo pathway-dependent necrosis resulting from mild cognitive impairment in brain neurons, and preventing decline in cognitive function and/or restoring declined cognitive functions. Hence, the present YAP-related substance is effective not only for the prevention or treatment of mild cognitive impairment but for the prevention of dementia (e.g., Alzheimer's dementia) to which mild cognitive impairment progresses.

### Brief Description of Drawings

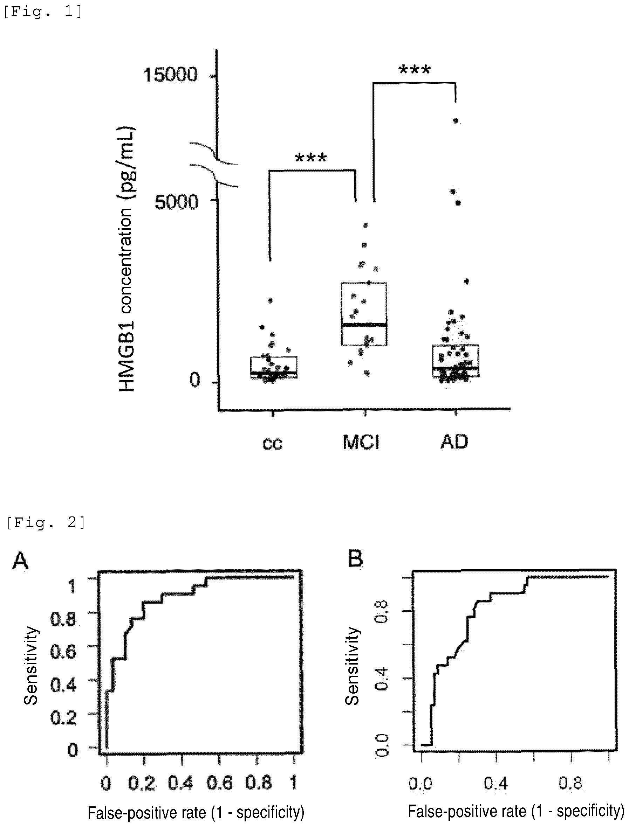

[Figure 1] Figure 1 is a diagram showing results of measuring HMGB1 concentrations in CSF samples derived from three types of test subjects (non-MCI/AD control subjects ["cc" in the drawing], MCI patients ["MCI" in the drawing], and AD patients ["AD" in the drawing]). In the drawing, "***" depicts statistically significant difference (p < 0.001) . In the drawing, • depicts each individual of the test subjects.
[Figure 2] Figure 2A is a diagram showing results of preparing a receiver operating characteristic (ROC) curve on the basis of results of measuring HMGB1 concentrations in CSF samples derived from MCI patients and non-MCI/AD control subjects. Figure 2B is a diagram showing results of preparing a ROC curve on the basis of results of measuring HMGB1 concentrations in CSF samples derived from MCI patients and AD patients.

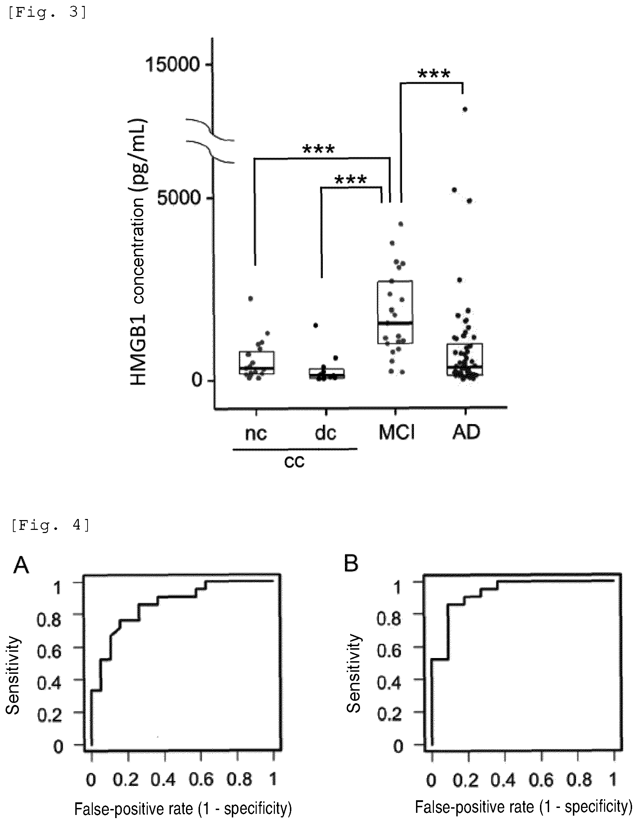

[Figure 3] Figure 3 is a diagram showing results of illustrating the non-MCI/AD control subjects of Figure 1 divided into two groups, healthy individuals ("nc" in the drawing) and disease patients ("dc" in the drawing).
[Figure 4] Figure 4A is a diagram showing results of preparing a ROC curve on the basis of results of measuring HMGB1 concentrations in CSF samples derived from MCI patients and healthy individuals. Figure 4B is a diagram showing results of preparing a ROC curve on the basis of results of measuring HMGB1 concentrations in CSF samples derived from MCI patients and non-dementia nervous disease patients.

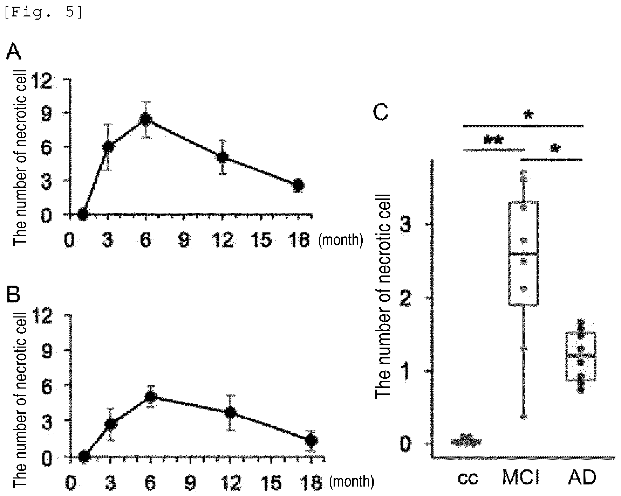

[Figure 5] Figure 5 is a diagram showing results of measuring the number of cells that necrosed (necrotic cell number) in the brain neurons of two types of AD mouse models (5×FAD mice [Figure 5A] and APP-KI mice [Figure 5B], n = 3 each) and the three types of test subjects. In the drawing, "*" and "**" depict statistically significant difference (p < 0.05 and p < 0.01, respectively) according to Tukey's HSD test.

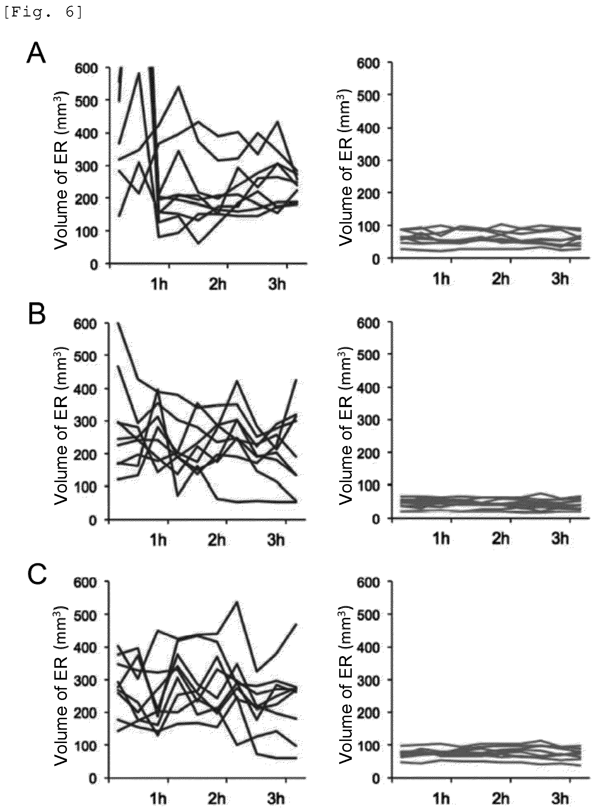

[Figure 6] Figure 6A is a diagram showing results of successively (1 to 3 hours) measuring endoplasmic reticulum (ER) volumes in the brain neurons (n = 9 per mouse) of 5×FAD mice (left graph, n = 3) and normal mice (right graph, n = 3) aged 1 month. Figure 6B is a diagram showing results of successively (1 to 3 hours) measuring ER volumes in the brain neurons (n = 9 per mouse) of 5×FAD mice (left graph, n = 3) and normal mice (right graph, n = 3) aged 3 months. Figure 6C is a diagram showing results of successively (1 to 3 hours) measuring ER volumes in the brain neurons (n = 9 per mouse) of 5×FAD mice (left graph, n = 3) and normal mice (right graph, n = 3) aged 6 months.

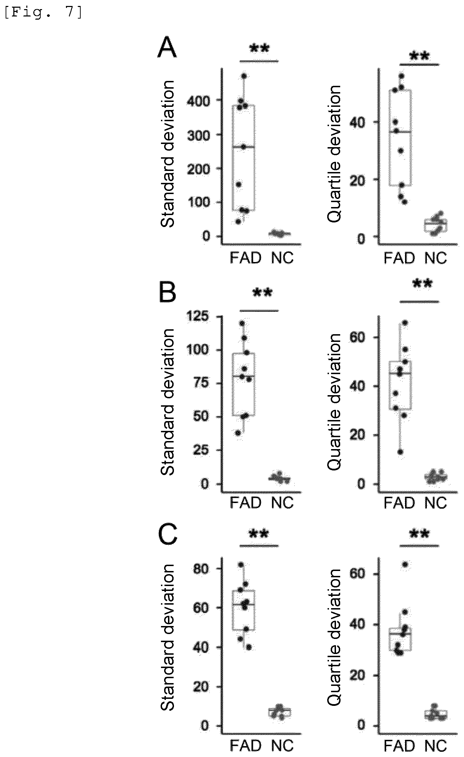

[Figure 7] Each of Figures 7A to 7C is a diagram showing results of measuring standard deviation (SD) (left graph) and quartile deviation (right graph) of the ER volumes on the basis of the results of Figures 6A to 6C, respectively. In the drawing, "**" depicts statistically significant difference (p < 0.01) according to Welch's test.

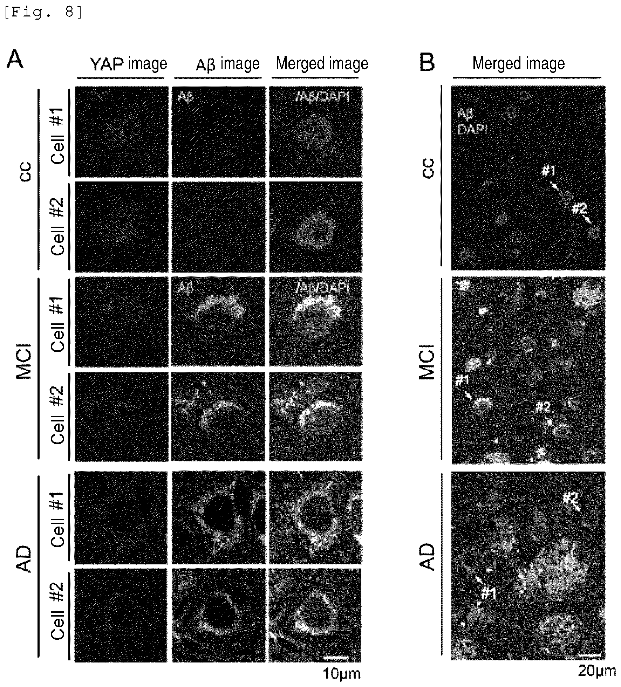

[Figure 8] Figure 8A shows YAP images, amyloid β (Aβ) images, and merged images (images in which the YAP image, the Aβ image, and a DAPI image were overlaid with each other) as to the brain neurons of the three types of test subjects. Figure 8B shows images on which the images of Figure 8A were based.

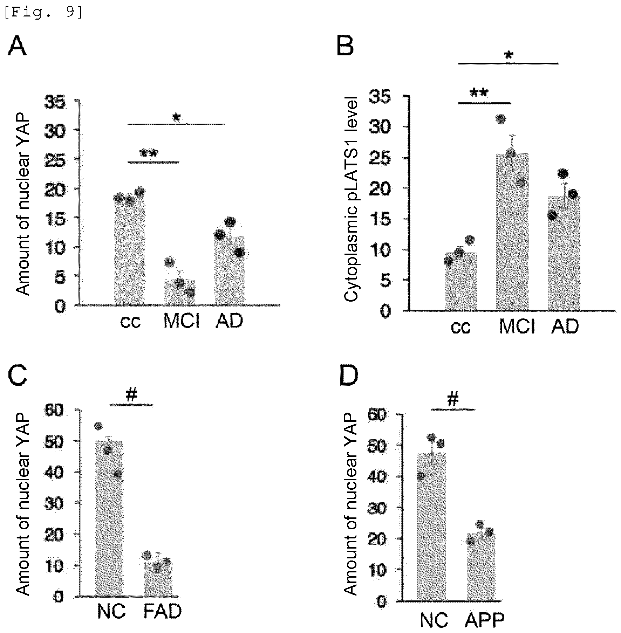

[Figure 9] Figure 9A is a diagram showing results of measuring nuclear YAP fluorescence levels (i.e., the amounts of nuclear YAP) on the basis of the YAP images of Figure 8 as to the brain neurons (n = 90 per test subject) of the three types of test subjects (n = 3 each). Figure 9B is a diagram showing results of measuring cytoplasmic pLATS1 levels as to the brain neurons (n = 90 per test subject) of the three types of test subjects (n = 3 each). Figure 9C is a diagram showing results of measuring the amounts of nuclear YAP as to the brain neurons (n = 90 per mouse) of 5×FAD mice ("FAD" in the drawing, n = 3) and normal mice ("NC" in the drawing, n = 3) aged 3 months. Figure 9D is a diagram showing results of measuring the amounts of nuclear YAP as to the brain neurons (n = 90 per mouse) of APP-KI mice ("APP" in the drawing, n = 3) and normal mice ("NC" in the drawing, n = 3) aged 3 months. In the drawing, "*" and "**" depict statistically significant difference (p < 0.05 and p < 0.01, respectively) according to Tukey's HSD test. In the drawing, "#" depicts statistically significant difference (p < 0.05) according to Student's t-test.

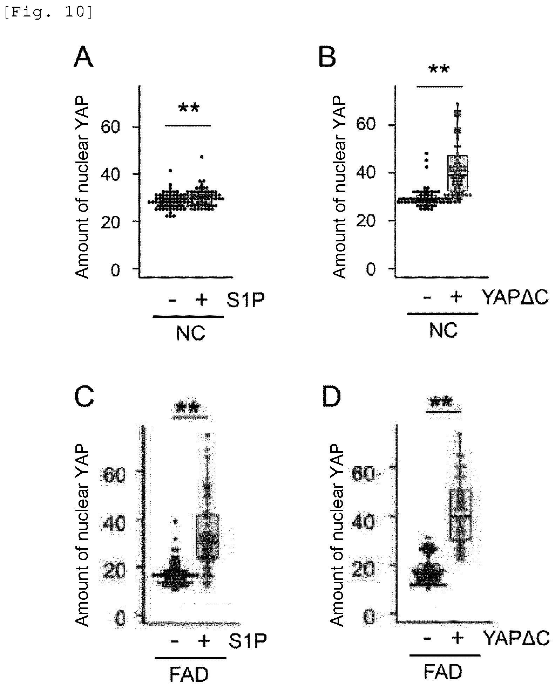

[Figure 10] Figure 10A is a diagram showing results of measuring the amounts of nuclear YAP as to the brain neurons (n = 60 per mouse) of normal mice ("+S1P NC" and "-S1P NC" in the drawing) continuously given S1P or given aCSF for a period from 1 to 6 months of age. Figure 10B is a diagram showing results of measuring the amounts of nuclear YAP as to the brain neurons (n = 60 per mouse) of normal mice ("+YAPΔC NC" and "-YAPΔC NC" in the drawing) given AAV-YAPΔC-ins61 or given AAV-NINS at 1 month of age. Figure 10C is a diagram showing results of measuring the amounts of nuclear YAP as to the brain neurons (n = 60 per mouse) of 5×FAD mice ("+S1P FAD" and "-S1P FAD" in the drawing) continuously given S1P or given aCSF for a period from 1 to 6 months of age. Figure 10D is a diagram showing results of measuring the amounts of nuclear YAP as to the brain neurons (n = 60 per mouse) of 5×FAD mice ("+YAPΔC FAD" and "-YAPΔC FAD" in the drawing) given AAV-YAPΔC-ins61 or given AAV-NINS at 1 month of age. In the drawing, "**" depicts statistically significant difference (p < 0.01) according to Welch's test.

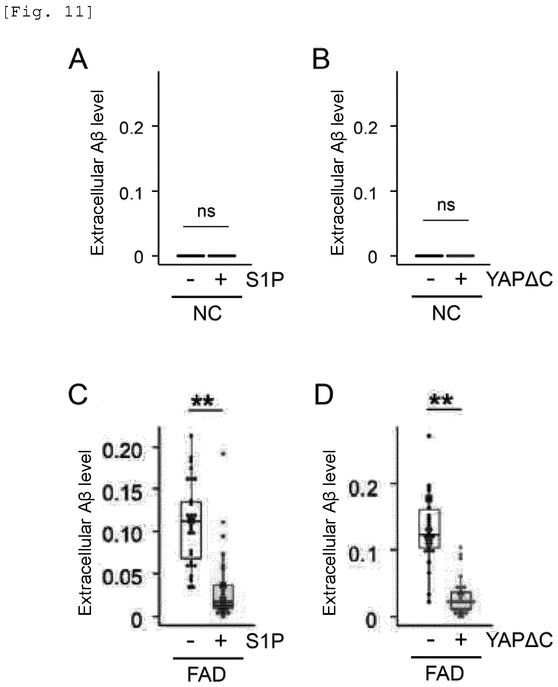

[Figure 11] Figure 11A is a diagram showing results of measuring extracellular Aβ levels as to the brain tissues (n = 30 per mouse) of normal mice ("+S1P NC" and "-S1P NC" in the drawing) continuously given S1P or given aCSF for a period from 1 to 6 months of age. Figure 11B is a diagram showing results of measuring extracellular Aβ levels as to the brain tissues (n = 30 per mouse) of normal mice ("+YAPΔC NC" and "-YAPΔC NC" in the drawing) given AAV-YAPΔC-ins61 or given AAV-NINS at 1 month of age. Figure 11C is a diagram showing results of measuring extracellular Aβ levels as to the brain tissues (n = 30 per mouse) of 5×FAD mice ("+S1P FAD" and "-S1P FAD" in the drawing) continuously given S1P or given aCSF for a period from 1 to 6 months of age. Figure 11D is a diagram showing results of measuring extracellular Aβ levels as to the brain tissues (n = 30 per mouse) of 5×FAD mice ("+YAPΔC FAD" and "-YAPΔC FAD" in the drawing) given AAV-YAPΔC-ins61 or given AAV-NINS at 1 month of age. In the drawing, "**" and "ns" depict statistically significant difference (p < 0.01) and no statistically significant difference (p ≥ 0.05), respectively, according to Welch's test.

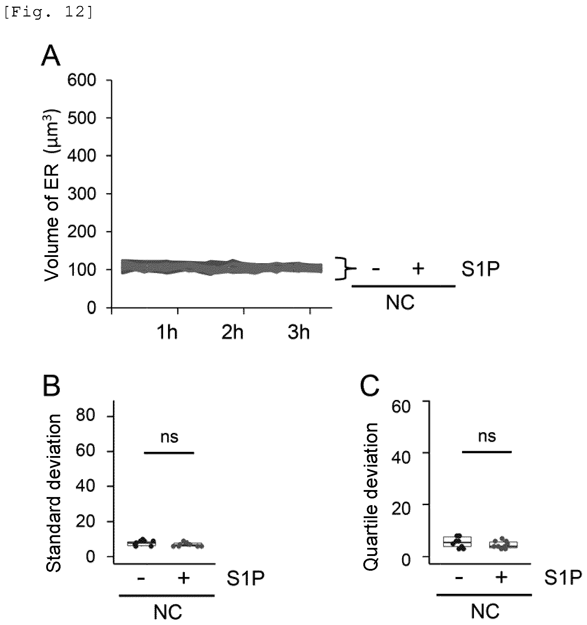

[Figure 12] Figure 12A is a diagram showing results of successively (1 to 3 hours) measuring ER volumes in the brain neurons (n = 3 per mouse) of normal mice ("+S1P NC" and "-S1P NC" in the drawing, n = 3) continuously given S1P or given aCSF for a period from 1 to 6 months of age. Each of Figures 12B and 12C is a diagram showing results of measuring standard deviation (Figure 12B) and quartile deviation (Figure 12C) of the ER volumes on the basis of the results of Figure 12A. In the drawing, "ns" depicts no statistically significant difference (p ≥ 0.05) according to Welch's test.

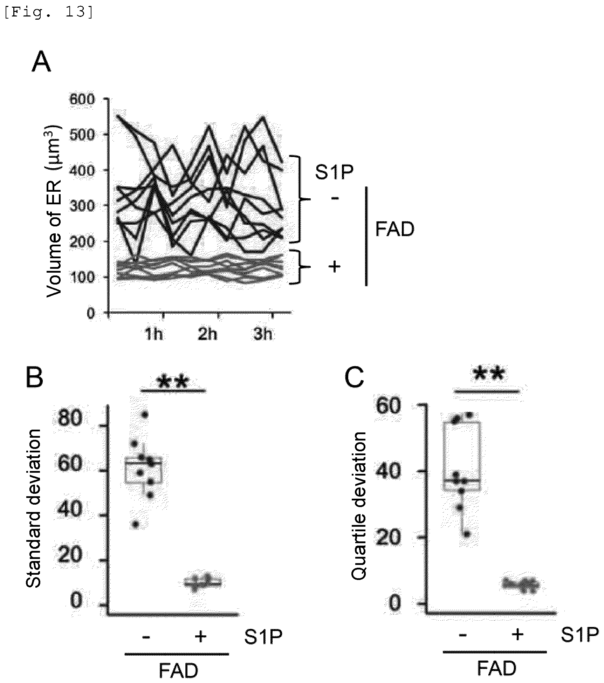

[Figure 13] Figure 13A is a diagram showing results of successively (1 to 3 hours) measuring ER volumes in the brain neurons (n = 3 per mouse) of 5×FAD mice ("+S1P FAD" and "-S1P FAD" in the drawing, n = 3) continuously given S1P or given aCSF for a period from 1 to 6 months of age. Each of Figures 13B and 13C is a diagram showing results of measuring standard deviation (Figure 13B) and quartile deviation (Figure 13C) of the ER volumes on the basis of the results of Figure 13A. In the drawing, "**" depicts statistically significant difference (p < 0.01) according to Welch's test.

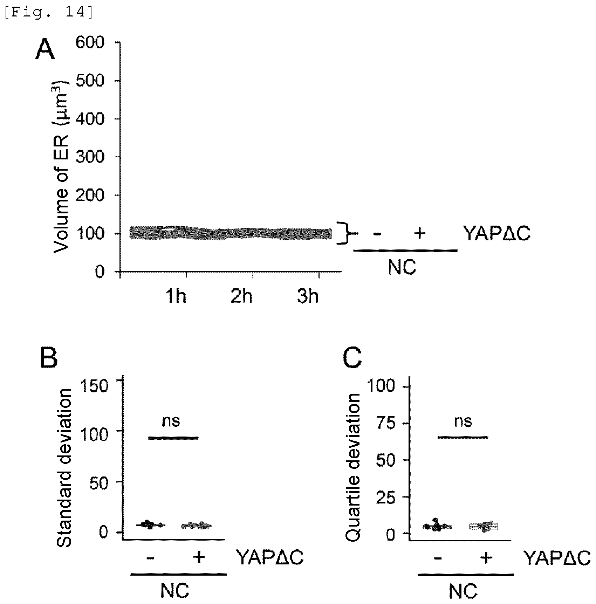

[Figure 14] Figure 14A is a diagram showing results of successively (1 to 3 hours) measuring ER volumes in the brain neurons (n = 3 per mouse) of normal mice ("+YAPΔC NC" and "-YAPΔC NC" in the drawing, n = 3) given AAV-YAPΔC-ins61 or given AAV-NINS at 1 month of age. Each of Figures 14B and 14C is a diagram showing results of measuring standard deviation (Figure 14B) and quartile deviation (Figure 14C) of the ER volumes on the basis of the results of Figure 14A. In the drawing, "ns" depicts no statistically significant difference (p ≥ 0.05) according to Welch's test.

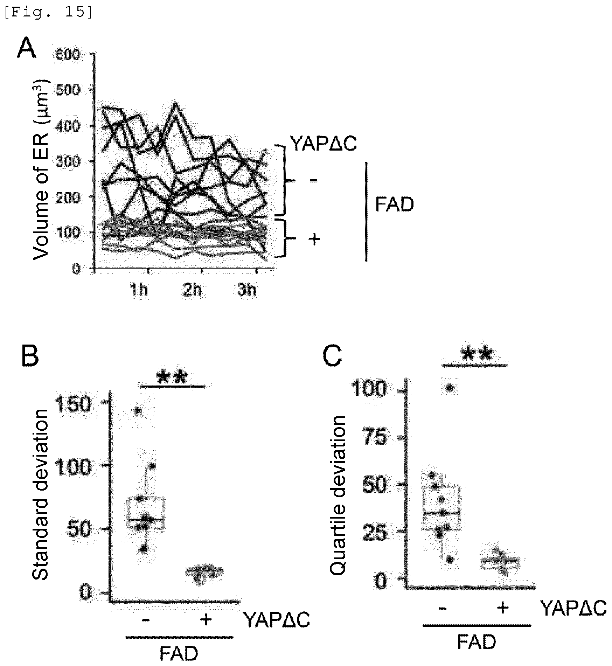

[Figure 15] Figure 15A is a diagram showing results of successively (1 to 3 hours) measuring ER volumes in the brain neurons (n = 3 per mouse) of 5×FAD mice ("+YAPΔC FAD" and "-YAPΔC FAD" in the drawing) given AAV-YAPΔCins61 or given AAV-NINS at 1 month of age. Each of Figures 15B and 15C is a diagram showing results of measuring standard deviation (Figure 15B) and quartile deviation (Figure 15C) of the ER volumes on the basis of the results of Figure 15A. In the drawing, "**" depicts statistically significant difference (p < 0.01) according to Welch's test.

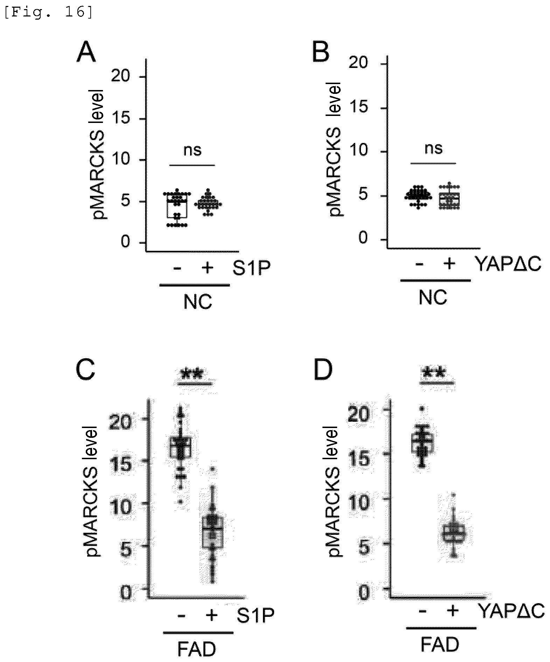

[Figure 16] Figure 16A is a diagram showing results of measuring pMARCKS levels as to the brain neurons (n = 30 per mouse) of normal mice ("+S1P NC" and "-S1P NC" in the drawing) continuously given S1P or given aCSF for a period from 1 to 6 months of age. Figure 16B is a diagram showing results of measuring pMARCKS levels as to the brain neurons (n = 30 per mouse) of normal mice ("+YAPΔC NC" and "-YAPΔC NC" in the drawing) given AAV-YAPΔC-ins61 or given AAV-NINS at 1 month of age. Figure 16C is a diagram showing results of measuring pMARCKS levels as to the brain neurons (n = 30 per mouse) of 5×FAD mice ("+S1P FAD" and "-S1P FAD" in the drawing) continuously given S1P or given aCSF for a period from 1 to 6 months of age. Figure 16D is a diagram showing results of measuring pMARCKS levels as to the brain neurons (n = 30 per mouse) of 5×FAD mice ("+YAPΔC FAD" and "-YAPΔC FAD" in the drawing) given AAV-YAPΔC-ins61 or given AAV-NINS at 1 month of age. In the drawing, "**" and "ns" depict statistically significant difference (p < 0.01) and no statistically significant difference (p ≥ 0.05), respectively, according to Welch's test.

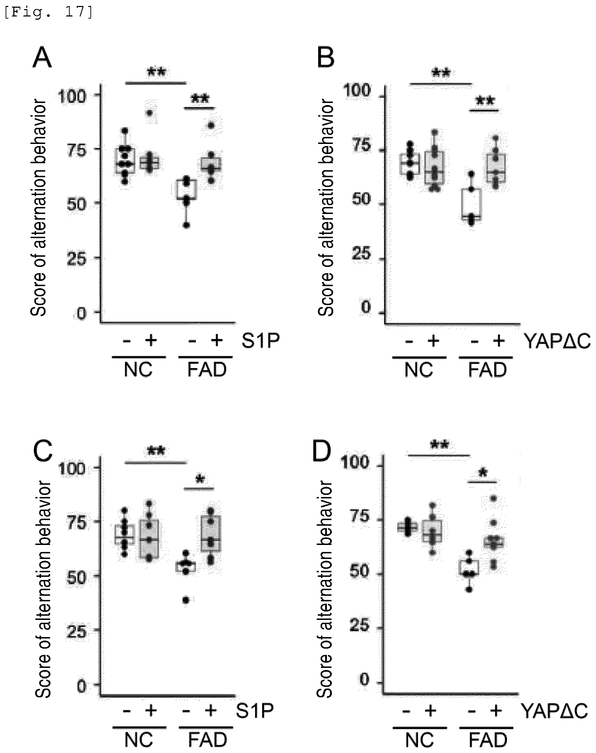

[Figure 17] Figure 17A is a diagram showing results of measuring the scores of alternation behavior as to 5×FAD mice ("+S1P FAD" and "-S1P FAD" in the drawing, n = 6 and 9) and normal mice ("+S1P NC" and "-S1P NC" in the drawing, n = 7 and 9) continuously given S1P or given aCSF for a period from 1 to 6 months of age. Figure 17B is a diagram showing results of measuring the scores of alternation behavior as to 5×FAD mice ("+YAPΔC FAD" and "-YAPΔC FAD" in the drawing, n = 7 and 5) and normal mice ("+YAPΔC NC" and "-YAPΔC NC" in the drawing, n = 10 and 8) given AAV-YAPΔC-ins61 or given AAV-NINS at 1 month of age. Figure 17C is a diagram showing results of measuring the scores of alternation behavior as to 5×FAD mice ("+S1P FAD" and "-S1P FAD" in the drawing, n = 7 and 7) and normal mice ("+S1P NC" and "-S1P NC" in the drawing, n = 7 and 8) continuously given S1P or given aCSF for a period from 5 to 6 months of age. Figure 17D is a diagram showing results of measuring the scores of alternation behavior as to 5×FAD mice ("+YAPΔC FAD" and "-YAPΔC FAD" in the drawing, n = 9 and 5) and normal mice ("+YAPΔC NC" and "-YAPΔC NC" in the drawing, n = 6 and 5) given AAV-YAPΔCins61 or given AAV-NINS at 5 months of age. In the drawing, "*" and "**" depict statistically significant difference (p < 0.05 and p < 0.01, respectively) according to Tukey's HSD test.

### Mode of Carrying Out the Invention

The agent for preventing or treating mild cognitive impairment according to the present invention is specified for the purpose of "preventing or treating mild cognitive impairment" and is a formulation (hereinafter, also referred to as the "present preventing/treating agent") comprising at least one member (preferably as an active ingredient) selected from mammalian YAP; a polynucleotide encoding mammalian YAP (hereinafter, also referred to as a "mammalian YAP polynucleotide"); and a substance capable of increasing an amount of mammalian YAP in a nucleus of a brain neuron (hereinafter, also referred to as a "mammalian YAP-enhancing substance"). In this context, the prevention of mild cognitive impairment includes the prevention of the development of mild cognitive impairment as well as the prevention of the symptomatic worsening of mild cognitive impairment. The present preventing/treating agent may be used alone as a medicament (formulation) or may be further mixed with an additive and used in the form of a composition (pharmaceutical composition).

Examples of the mammal can include: a rodent such as a mouse, a rat, a hamster, and a guinea pig; a lagomorph such as a rabbit; an animal of the order *Artiodactyla* such as a pig, a bovine, a goat, a horse, and sheep; an animal of the order *Carnivora* such as a dog and a cat; and a primate such as a human, a monkey, a rhesus macaque, a cynomolgus, a marmoset, an orangutan, and a chimpanzee.

The mammalian YAP can be mammalian YAP, i.e., a mammal-derived polypeptide that is localized in a nucleus of a brain neuron and has action of suppressing Hippo pathway-dependent necrosis caused by mild cognitive impairment. Examples thereof can include a polypeptide comprising an amino acid sequence having at least 80% sequence identity to the amino acid sequence of rat YAPΔCins61 (i.e., the amino acid sequence shown by SEQ ID NO: 1), a polypeptide comprising an amino acid sequence having at least 80% sequence identity to the amino acid sequence of rat YAPΔC-ins13 (i.e., the amino acid sequence shown by SEQ ID NO: 3), a polypeptide comprising an amino acid sequence having at least 80% sequence identity to the amino acid sequence of rat YAPΔCs-ins25 (i.e., the amino acid sequence shown by SEQ ID NO: 4), a polypeptide comprising an amino acid sequence having at least 80% sequence identity to the amino acid sequence of rat YAP (i.e., the amino acid sequence shown by SEQ ID NO: 5), a polypeptide comprising an amino acid sequence having at least 80% sequence identity to the amino acid sequence of mouse YAP (i.e., the amino acid sequence shown by SEQ ID NO: 6), a polypeptide comprising an amino acid sequence having at least 80% sequence identity to the amino acid sequence of human YAP2 (i.e., the amino acid sequence shown by SEQ ID NO: 7), a polypeptide comprising an amino acid sequence having at least 80% sequence identity to the amino acid sequence of human YAP1-2 delta (i.e., the amino acid sequence shown by SEQ ID NO: 8), a polypeptide comprising an amino acid sequence having at least 80% sequence identity to the amino acid sequence of human YAP1-2 alpha (i.e., the amino acid sequence shown by SEQ ID NO: 9), a polypeptide comprising an amino acid sequence having at least 80% sequence identity to the amino acid sequence of human YAP1-2 beta (i.e., the amino acid sequence shown by SEQ ID NO: 10), a polypeptide comprising an amino acid sequence having at least 80% sequence identity to the amino acid sequence of human YAP1-2 gamma (i.e., the amino acid sequence shown by SEQ ID NO: 11), a polypeptide comprising an amino acid sequence having at least 80% sequence identity to the amino acid sequence of human YAP1 (i.e., the amino acid sequence shown by SEQ ID NO: 12), a polypeptide comprising an amino acid sequence having at least 80% sequence identity to the amino acid sequence of human YAP1-1 gamma (i.e., the amino acid sequence shown by SEQ ID NO: 13), a polypeptide comprising an amino acid sequence having at least 80% sequence identity to the amino acid sequence of human YAP1-1 delta (i.e., the amino acid sequence shown by SEQ ID NO: 14), a polypeptide comprising an amino acid sequence having at least 80% sequence identity to the amino acid sequence of human YAP1-1 alpha (i.e., the amino acid sequence shown by SEQ ID NO: 15), and a polypeptide comprising an amino acid sequence having at least 80% sequence identity to the amino acid sequence of human YAP1-1 beta (i.e., the amino acid sequence shown by SEQ ID NO: 16). Preferred examples thereof can include a polypeptide comprising an amino acid sequence having at least 80% sequence identity to the amino acid sequence of rat YAPΔC-ins61 (i.e., the amino acid sequence shown by SEQ ID NO: 1) because its effect has been demonstrated in the present Examples mentioned later. The polypeptide comprising an amino acid sequence having at least 80% sequence identity to the amino acid sequence shown by SEQ ID NO: 1 encompasses rat YAPΔC-ins61 (polypeptide consisting of the amino acid sequence shown by SEQ ID NO: 1) as well as rat YAPΔC-ins13 (polypeptide consisting of the amino acid sequence shown by SEQ ID NO: 3), rat YAPΔC-ins25 (polypeptide consisting of the amino acid sequence shown by SEQ ID NO: 4), rat YAP (polypeptide consisting of the amino acid sequence shown by SEQ ID NO: 5), mouse YAP (polypeptide consisting of the amino acid sequence shown by SEQ ID NO: 6), human YAP2 (polypeptide consisting of the amino acid sequence shown by SEQ ID NO: 7), human YAP1-2 delta (polypeptide consisting of the amino acid sequence shown by SEQ ID NO: 8), human YAP1-2 alpha (polypeptide consisting of the amino acid sequence shown by SEQ ID NO: 9), human YAP1-2 beta (polypeptide consisting of the amino acid sequence shown by SEQ ID NO: 10), human YAP1-2 gamma (polypeptide consisting of the amino acid sequence shown by SEQ ID NO: 11), and the like.

The number of amino acid residues of the mammalian YAP is not particularly limited. The number of amino acid residues of the human, mouse, and rat YAP (i.e., the amino acid sequences shown by SEQ ID NOs: 1 and or 3 to 16) mentioned above falls within the range of 350 to 508. The number of amino acid residues of the rat YAPΔC-ins61 specifically used in the present Examples mentioned later is 366. In consideration of these, examples thereof can include 280 to 700, 280 to 660, 280 to 630, 280 to 600, 280 to 560, 280 to 530, 280 to 510, 290 to 700, 300 to 700, 310 to 700, 320 to 700, 330 to 700, 340 to 700, 350 to 700, 366 to 700, 290 to 700, 290 to 660, 290 to 630, 290 to 600, 290 to 560, 290 to 530, 290 to 510, 300 to 700, 300 to 660, 300 to 630, 300 to 600, 300 to 560, 300 to 530, 300 to 510, 310 to 700, 310 to 660, 310 to 630, 310 to 600, 310 to 560, 310 to 530, 310 to 510, 320 to 700, 320 to 660, 320 to 630, 320 to 600, 320 to 560, 320 to 530, 320 to 510, 330 to 700, 330 to 660, 330 to 630, 330 to 600, 330 to 560, 330 to 530, 330 to 510, 340 to 700, 340 to 660, 340 to 630, 340 to 600, 340 to 560, 340 to 530, 340 to 510, 350 to 700, 350 to 660, 350 to 630, 350 to 600, 350 to 560, 350 to 530, 350 to 510, 360 to 700, 360 to 660, 360 to 630, 360 to 600, 360 to 560, 360 to 530, and 360 to 510. The amino acid residues of the mammalian YAP may be modified by glycosylation, acetylation, phosphorylation, lipidation, stable isotope labeling, or the like.

As for the mammalian YAP polynucleotide, those skilled in the art can specifically and clearly understand a nucleotide sequence corresponding to the amino acid sequence thereof by referring to the amino acid sequence of the mammalian YAP and a codon table known in the art. When the mammalian YAP is, for example, a polypeptide comprising an amino acid sequence having at least 80% sequence identity to the amino acid sequence of rat YAPΔCins61 (i.e., the amino acid sequence shown by SEQ ID NO: 1), examples thereof can include a polynucleotide comprising a nucleotide sequence having at least 80% sequence identity to the nucleotide sequence of cDNA (polynucleotide) encoding rat YAPΔC-ins61 (i.e., a polynucleotide consisting of the nucleotide sequence shown by SEQ ID NO: 2).

Preferred examples of the mammalian YAP polynucleotide can include a vector comprising a polynucleotide having a promoter operably linked upstream of the mammalian YAP polynucleotide. The vector is not particularly limited and can be appropriately selected for any purpose as long as the vector permits transcription of mRNA encoded by the mammalian YAP polynucleotide. Examples thereof can include a non-viral vector (e.g., an episomal vector, an artificial chromosome vector, and a plasmid vector) and a virus vector. The vector may be circular or may be linear.

The promoter for use in the vector is not particularly limited as long as the region undergoes the binding of RNA polymerase (preferably RNA polymerase and a basal transcription factor) and triggers the transcription of mRNA encoded by the mammalian YAP polynucleotide positioned downstream thereof. Examples thereof can include SRα promoter, SV40 early promoter, retrovirus LTR, CMV (cytomegalovirus) promoter, RSV (Rous sarcoma virus) promoter, HSV-TK (herpes simplex virus thymidine kinase) promoter, EF1α promoter, metallothionein promoter, and heat shock promoter. The promoter may be used together with an enhancer of human CMV IE gene. As one example, CAG promoter (comprising cytomegalovirus enhancer, chicken β-actin promoter, and a poly A signal site of β-globin gene) can be used.

The episomal vector is a vector capable of replicating autonomously outside the chromosome. A specific approach using the episomal vector is disclosed in Yu et al., Science, 324, 797-801 (2009). In a particularly preferred embodiment of the present invention, an episomal vector having loxP sequences located in the same orientation on the 5' side and 3' side of a vector element necessary for the replication of the episomal vector can be used. The episomal vector, which is capable of replicating autonomously outside the chromosome, is capable of providing stable expression in host cells without being integrated into the genome.

Examples of the episomal vector can include a vector comprising, as a vector element, a sequence necessary for autonomous replication derived from EBV, SV40, or the like. The vector element necessary for autonomous replication is specifically a replication origin and a gene encoding a protein that binds to the replication origin and controls replication. Examples thereof can include a replication origin oriP and EBNA-1 gene for EBV, and a replication origin ori and SV40LT gene for SV40.

Examples of the artificial chromosome vector can include YAC (yeast artificial chromosome) vector, BAC (bacterial artificial chromosome) vector, and PAC (P1-derived artificial chromosome) vector.

Examples of the plasmid vector can include pA1-11, pXT1, pRc/CMV, pRc/RSV, and pcDNAI/Neo.

The virus vector means a gene vector that exploits the infectious ability or replicative ability of a virus, and more specifically means a virus particle (also referred to as a "recombinant virus") containing a virus vector plasmid (which may be DNA or may be RNA) obtained by removing a gene associated with pathogenicity from the virus genome and inserting a foreign gene (in the present application, the mammalian YAP polynucleotide). Examples of the virus vector can include a retrovirus vector, a lentivirus vector, an adenovirus vector, an AAV (adenoassociated virus) vector, a Sendai virus vector, a herpes virus vector, a vaccinia virus vector, a pox virus vector, a polio virus vector, a Sindbis virus vector, rhabdovirus vector, paramyxovirus vector, and orthomyxovirus vector. An adenovirus vector or an AAV vector is preferred because the introduction efficiency of the mammalian YAP polynucleotide to non-dividing cells such as brain neurons is relatively high. A lentivirus vector or an AAV vector is preferred because the expression of mammalian YAP can be sustained for a long period in brain neurons after introduction of the mammalian YAP polynucleotide. An AAV vector is preferred because of being non-pathogenic and highly safe. Thus, preferred examples of the virus vector can include an AAV vector because the introduction efficiency of the mammalian YAP polynucleotide to non-dividing cells such as brain neurons is relatively high, because the expression of mammalian YAP can be sustained for a long period in brain neurons after introduction of the mammalian YAP polynucleotide, and because of being non-pathogenic and highly safe.

The AAV vector comprising the mammalian YAP polynucleotide is produced in the nuclei, for example, by transfecting HEK293 cells comprising genes (rep gene and cap gene) encoding AAV proteins necessary for the replication of AAV and the formation of AAV particles, and genes (E2A gene, E4 gene, and VARNA gene) encoding proteins necessary for the proliferation of AAV with the AAV vector comprising the mammalian YAP polynucleotide, and recovered for separation and/or purification from the nuclei by a method such as a density gradient centrifugation method using cesium chloride or a column method after freezing and thawing (documents: "Li, X. G. et al. Mol Ther 13, 160-166 (2006)", "Matsushita, T. et al. Gene Ther 5: 938-945 (1998)", and "Urabe, M. et al. Mol Ther 13: 823-828 (2006)").

The vector may contain, in addition to the promoter, an enhancer, a poly A addition signal, a marker gene, a replication origin, a gene encoding a polypeptide that binds to the replication origin and controls replication, and the like, if desired. The marker gene refers to a gene that permits screening or selection of cells by introducing the marker gene to the cells. Specific examples of the marker gene can include a drug resistance gene, a fluorescent protein gene, a luminescent enzyme gene, and a chromogenic enzyme gene. One of these marker genes may be used singly, or two or more thereof may be used in combination. Specific examples of the drug resistance gene can include neomycin resistance gene, tetracycline resistance gene, kanamycin resistance gene, zeocin resistance gene, and hygromycin resistance gene. Specific examples of the fluorescent protein gene can include green fluorescent protein (GFP) gene, yellow fluorescent protein (YFP) gene, and red fluorescent protein (RFP) gene. Specific examples of the luminescent enzyme gene can include luciferase gene. Specific examples of the chromogenic enzyme gene can include β galactosidase gene, β glucuronidase gene, and alkaline phosphatase gene.

The mammalian YAP polynucleotide may be DNA or may be RNA, or may be a DNA/RNA chimera. DNA is preferred from the viewpoint of stability. The polynucleotide may be partially or wholly substituted by an artificial nucleotide such as PNA (polyamide nucleic acid), LNA(R) (locked nucleic acid or bridged nucleic acid), ENA(R) (2'-O,4'-C-ethylene-bridged nucleic acids), GNA (glycerol nucleic acid), or TNA (threose nucleic acid). The mammalian YAP polynucleotide may be a single-stranded (sense strand) polynucleotide encoding mammalian YAP or may be a double-stranded polynucleotide consisting of the sense strand and an antisense strand having a complementary sequence thereof. A double-stranded polynucleotide is preferred.

In the present specification, the "at least 80% sequence identity to the amino acid sequence ..." means that the percentage of amino acids identical to those of the amino acid sequence to be compared is 80% or higher, and means preferably 85% or higher, more preferably 88% or higher, further preferably 90% or higher, still further preferably 93% or higher, particularly preferably 95% or higher, particularly more preferably 98% or higher, most preferably 99% sequence identity. In the present specification, the "at least 80% sequence identity to the nucleotide sequence ..." means that the percentage of nucleotides identical to those of the nucleotide sequence to be compared is 80% or higher, and means preferably 85% or higher, more preferably 88% or higher, further preferably 90% or higher, still further preferably 93% or higher, particularly preferably 95% or higher, particularly more preferably 98% or higher, most preferably 99% sequence identity. The identity of the amino acid sequence or the nucleotide sequence can be determined using a program known in the art such as ClustalW, GENETYX, or BLAST.

The mammalian YAP-enhancing substance is not particularly limited as long as the substance is capable of increasing an amount of mammalian YAP in a nucleus of a brain neuron by an action of promoting the translocation of the mammalian YAP into a brain neuron nucleus, action of enhancing the expression of the mammalian YAP in a brain neuron nucleus, or the like (e.g., a polypeptide such as an antibody; a polynucleotide; a saccharide; a lipid; an organic compound; and an inorganic compound). Examples thereof can include a lysophospholipid (e.g., lysophosphatidic acid [LPA], sphingosine 1-phosphate[S1P], lysophosphatidylserine [LPS], lysophosphatidylinositol [LPI], and lysophosphatidylethanolamine [LPE]), and a S1P receptor agonist (e.g., KRP-203 [2-amino-2-[2-[2-chloro-4-[[3-(phenylmethoxy)phenyl]thio]phenyl]ethyl]-1,3-propanediol], CS-0777 [1-[5-[(3R)-3-amino-4-hydroxy-3-methylbutyl]-1-methylpyrrol-2-yl]-4-(4-methylphenyl)butan-1-one], ponesimod [(2Z,5Z)-5-(3-chloro-4-((R)-2,3-dihydroxypropoxy)benzylidene)-2-(propyliMino)-3-(o-tolyl)thiazolidin-4-one], and siponimod [1-(4-[1-[(E)-4-cyclohexyl-3-trifluoromethyl-benzyloxyimino]-ethyl]-2-ethyl-benzyl)-azetidine-3-carboxylicacid]). Preferred examples thereof can include S1P because its effect has been demonstrated in the present Examples mentioned later.

The present preventing/treating agent is administered to a subject in need of the prevention or treatment of mild cognitive impairment. The method for administering the present preventing/treating agent to the subject can be appropriately selected in consideration of the form of the present YAP-related substance. When the present YAP-related substance is, for example, mammalian YAP or a mammalian YAP polynucleotide, examples thereof can include parenteral administration (e.g., intravenous administration and administration into cerebrospinal fluid space by lumbar puncture, etc.). When the present YAP-related substance is a mammalian YAP-enhancing substance (preferably a low-molecular substance), examples thereof can include parenteral administration (e.g., intravenous administration, administration into cerebrospinal fluid space by lumbar puncture, etc., subcutaneous administration, intramuscular administration, and intravenous administration) and oral administration.

In the present invention, the "mild cognitive impairment" means a condition that has decline in cognitive function (e.g., a memory function, an executive function, an attentional function, a visual spatial cognitive function, and a language understanding function), which however does not satisfy the diagnostic criteria of dementia (in other words, has no dementia), and finds no problem in fundamental daily life or social life; and/or a condition having an elevated concentration of HMGB1 in cerebrospinal fluid as compared with a control subject having no mild cognitive impairment. In this context, the phrase "have decline in cognitive function, which however does not satisfy the diagnostic criteria of dementia" means that the score of MMSE (mini mental state examination) falls within the range of 19 to 27 (e.g., 20 to 27, 21 to 27, 22 to 27, 23 to 27, 24 to 27, 19 to 26, 19 to 25, 19 to 24, 19 to 23, 21 to 26, 21 to 25, 21 to 24, and 21 to 23); and/or the score of CDR (clinical dementia rating) is 0.5.

In the present specification, examples of the dementia can include Alzheimer's disease (Alzheimer's dementia), dementia that develops in Parkinson's disease, Lewy body dementia, frontotemporal dementia, dementia that develops in progressive non-fluent aphasia, semantic dementia, and dementia that develops in corticobasal degeneration.

In the present specification, the "control subject having no mild cognitive impairment" can be an individual having no mild cognitive impairment (a control of a test subject), i.e., an individual who satisfies at least one of the following criteria: 1) no decline in cognitive function is found; 2) the diagnostic criteria of dementia are satisfied; and 3) there is a problem in fundamental daily life or social life. Examples thereof can specifically include a healthy individual and a patient having a disease (e.g., one or more diseases selected from Alzheimer's disease, spinal conus syndrome, neuromyelitis optica, paraneoplastic syndrome, idiopathic normal pressure hydrocephalus, alcoholism, peripheral neuropathy, multiple sclerosis, systemic lupus erythematosus, lung cancer, and herpes zoster meningitis) other than mild cognitive impairment, and can preferably include a healthy individual and an Alzheimer's disease patient.

Hereinafter, the present invention will be described more specifically with reference to Examples. However, the technical scope of the present invention is not limited by these examples.

### Examples

### 1. Material and method

### [Preparation of CSF sample]

CSF was collected by lumbar puncture from 30 non-MCI/AD control subjects (19 healthy individuals and 11 disease patients), 21 MCI patients, and 56 AD patients among three types of test subjects (individuals having neither MCI nor AD [non-MCI/AD control subjects], MCI patients, and AD patients) shown in Table 1, and centrifuged (1000 × g, 10 min, 4°C) for the removal of foreign substances. The supernatants were preserved as CSF samples at -80°C. Whether or not the test subjects had MCI and AD was diagnosed according to the criteria defined in the International Classification of Diseases, Tenth Revision (ICD-10) provided by the World Health Organization. The test subjects diagnosed with MCI were test subjects who had decline in cognitive function, which however did not satisfy the diagnostic criteria of dementia, and found no problem in fundamental daily life.

### [Measurement of Aβ and pTau in CSF sample]

Two types of Aβ (Aβ40 and Aβ42) concentrations in the CSF samples were measured using an ELISA kit for detecting human Aβ40 (292-62301, manufactured by FUJIFILM Wako Pure Chemical Corp.) and an ELISA kit for detecting human Aβ42 (298-62401, manufactured by FUJIFILM Wako Pure Chemical Corp.). pTau concentrations in the CSF samples were measured using INNOTEST PHOSPHO-TAU (181P) (manufactured by Innogenetics N.V.).

### [Measurement of HMGB1 in CSF sample]

Each well of a polystyrene microtiter plate (manufactured by Nunc/Thermo Fisher Scientific Inc.) was coated with 100 µL of PBS containing 1 mg/L anti-human HMGB1 monoclonal antibody (2D4, manufactured by Shino-Test Corp.), and incubated overnight under a condition of 2 to 8°C. Then, each well was washed three times with PBS containing 0.05% Tween 20, blocked with 400 µL/well of PBS containing 1% BSA, and washed three times with PBS containing 0.05% Tween 20. Then, 100 µL of each CSF sample was added to each well, which was then incubated at 37°C for 24 hours. Then, each well was washed three times with PBS containing 0.05% Tween 20, and 100 µL of PBS containing 0.5 mg/L anti-human HMGB1,2 monoclonal antibody (R04, manufactured by Shino-Test Corp.) conjugated with peroxidase was added to each well, which was then incubated at room temperature for 2 hours. Each well was washed three times with PBS containing 0.05% Tween 20, and a chromogenic substrate TMB (3,3',5,5'-tetra-methyl benzidine) (manufactured by Dojindo Laboratories Co., Ltd.) was added to each well. The reaction treatment of TMB was terminated by adding 0.35 M sodium sulfate to each well. Absorbance at the maximum absorption wavelength of 450 nm was measured using a microplate reader, model 680 (manufactured by Bio-Rad Laboratories, Inc.). A standard curve of the absorbance was prepared using purified swine thymus gland-derived HMGB1 (manufactured by Shino-Test Corp.).

### [Preparation of brain tissue sample]

Brain tissues were isolated from 5 non-MCI/AD control subjects, 8 MCI patients, and 8 AD patients among the three types of test subjects shown in Table 1, and paraffin sections of the brain tissues were prepared according to a standard method. Use of the human brain tissue samples from the patients was approved by the ethical review board of Tokyo Medical and Dental University.

**[Table 1]**

| | Non-MCI/AD control subject | | MCI patient | AD patient |
|---|---|---|---|---|
| | Healthy subject | Disease patient | | |
| The number of persons | 34 | 14 | 26 | 73 |
| Male (%) | 21 (62%) | 8 (57%) | 14 (54%) | 35 (48%) |
| Female (%) | 13 (38%) | 6 (43%) | 12 (46%) | 38 (52%) |
| Age | 73±7.6 | 71±11 | 75±6.8 | 75±8.4 |
| MMSE value | 28±1.6 | 27±2.3 | 22±3.7 | 18±7.0 |
| CDR value | 0 | 0 | 0.5 | 1.5 |

In the table, "Age", "MMSE value", and "CDR value" are indicated in mean ± standard deviation (S.D.). The 14 disease patients are specifically one spinal conus syndrome patient, one neuromyelitis optica patient, one paraneoplastic syndrome patient, four idiopathic normal pressure hydrocephalus patients, one brain stem tumor patient, one patient having idiopathic normal pressure hydrocephalus and alcoholism, one peripheral neuropathy patient, one multiple sclerosis patient, one systemic lupus erythematosus patient, one patient having lung cancer and idiopathic normal pressure hydrocephalus, and one herpes zoster meningitis patient.

### [AD mouse model]

5×FAD transgenic B6/SJL mice (hereinafter, referred to as "5×FAD mice"), i.e., AD mouse models over-expressing human mutant APP (KM670/671NL Sweden mutation, 1716V Florida mutation, and V717I London mutation) and human mutant PS1 (M146L mutation and L285V mutation) (see the document "J Neurosci 26, 10129-10140 (2006)") were purchased from The Jackson Laboratory (Bar Harbor, Maine, USA). APP-KI mice, i.e., AD mouse models having human mutant APP (KM670/671NL Sweden mutation, E693G Arctic mutation, and I716F Beyreuther/Iberian mutation) (see the document "Nat Neurosci 17, 661-663 (2014)") were obtained from Riken BioResource Research Center.

### [Immunohistochemical staining method of brain tissue]

Brain tissues were excised from the test subjects or the AD mouse models and fixed in a 4% PFA (paraformaldehyde) solution. Then, 5 µm thick paraffin sections were prepared using a microtome (manufactured by Yamato Kohki Industrial Co., Ltd.). The cell nuclei were stained in PBS containing 0.2 µg/mL DAPI (4',6-diamidino-2-phenylindole) by an immunohistochemical staining method using four types of primary antibodies (rabbit antipSer46-MARCKS antibody [commissioning-manufactured by Biologica Co., Ltd., dilution ratio: 1:2000], mouse antiamyloid β antibody [clone 82E1, #10323, manufactured by Immuno-Biological Laboratories Co., Ltd., dilution ratio: 1:5000 or 1:1000], rabbit anti-pSer909-LATS1 antibody [#9157, manufactured by Cell Signaling Technology, Inc., dilution ratio: 1:100], and rabbit anti-YAP antibody [sc-15407, manufactured by Santa Cruz Biotechnology Inc., dilution ratio: 1:100]) and two types of secondary antibodies (Alexa 488-labeled donkey anti-mouse IgG [A-21202, manufactured by Molecular Probes, Inc., dilution ratio: 1:1000] and Alexa 568-labeled donkey anti-rabbit IgG [#A-10042, manufactured by Molecular Probes, Inc., dilution ratio: 1:1000]) according to a standard method. Fluorescence signals derived from each primary antibody were detected using a fluorescence microscope (IX70, manufactured by Olympus Corp.) or a confocal laser scanning microscope (FV1200, manufactured by Olympus Corp.) to obtain images (specifically, YAP images, phosphorylated LATS1 serine 909 (Ser909) [pLATS1] images, images of the detected DAPI fluorescence signals derived from the cells nuclei (DAPI images), and overlaid images thereof (merged images). In the DAPI images, cells having the deformed and/or shrunk cell nuclei were evaluated as necrotic cells, and the numbers of necrotic cells in 30 fields of view (143 µm × 143 µm) were measured to calculate a necrotic cell number per field of view (see the ordinates of Figures 5A to 5C).

### [In vivo imaging of ER and amyloid β in brain neuron]

The skull bones on RSD surface of normal mice (non-transgenic B6/SJL mice) or 5×FAD mice were removed using a high-speed microdrill. 1 µL of a liquid containing 100 nM amyloid-binding substance BTA1 (2-(4'-(methylamino)phenyl) benzothiazole, manufactured by Sigma-Aldrich Co. LLC) and 100 nM ER-Tracker Red (E34250, manufactured by Thermo Fisher Scientific Inc.) was injected to RSD in the presence of 1% isoflurane (anesthetic). 4 hours later, ER-Tracker Red was imaged using an upright microscope (BX61WI, manufactured by Olympus Corp.) and a scanning laser microscope system FV1000MPE2 (manufactured by Olympus Corp.) equipped with an immersion objective lens (XLPlanN25xW, numerical aperture: 1.05) and a pulse laser (MaiTaiHP DeepSee, manufactured by Spectra-Physics, Inc.) to obtain images of detected BTA1 fluorescence signals (BTA1 images) and images of detected ER-Tracker Red fluorescence signals (ER-Tracker images). The fluorescence signal volumes of ER-Tracker Red (i.e., volumes of ER) were measured.

### [Preparation of recombinant AAV]

Recombinant AAV (hereinafter, also referred to as "AAV-YAPΔC-ins61") expressing rat YAPΔC-ins61 (GenBank: ABA33617) (i.e., a polypeptide consisting of the amino acid sequence shown by SEQ ID NO: 1) was prepared according to the method described in the document "Li, X. G. et al., Mol Ther 13, 160-166 (2006)" by introducing cDNA encoding rat YAPΔC-ins61 (GenBank: DQ186898) (i.e., cDNA consisting of the nucleotide sequence shown by SEQ ID NO: 2) to an AAV vector. The prepared rAAV was purified by the density gradient centrifugation method of cesium chloride, and the titer of the virus was measured by use of quantitative PCR. Recombinant AAV (hereinafter, also referred to as "AAV-NINS") was prepared as a control using an AAV vector free from the cDNA.

### [Administration of rAAV and sphingosine 1-phosphate (S1P) to retrosplenial region]

The administration of AAV-YAPΔC-ins61 or AAV-NINS (titer: 1 × 10¹⁰ vector genomes/mL each, 1 µL) to mouse RSD (retrosplenial cortex) (depth of -2.0 mm from the bregma and 0.6 mm mediolaterally) and the subarachnoidal administration of S1P (40 nM, 0.15 µL/hr; manufactured by Sigma-Aldrich Co. LLC) or control artificial cerebrospinal fluid (aCSF) (0.15 µL/hr; manufactured by Alzet Osmotic Pressures) to the mice were performed according to the method described in the document "Yang, G. et al., Nat Protoc 5, 201-208 (2010)". The administration of AAV-YAPΔC-ins61 or AAV-NINS to the mice was performed at 1 or 5 months of age. The administration of S1P to the mice was continuously performed for a period from 1 to 6 months of age or for a period from 5 to 6 months of age.

### [Cognitive function analysis]

The normal mice (non-transgenic B6/SJL mice) or the 5×FAD mice given AAV-YAPΔC-ins61 or AAV-NINS, or S1P were tested for their exploratory behavior when aged 6 months using a Y-shaped maze (manufactured by O'HARA & Co., Ltd.) in which three arms of the same size were joined at 120°C. Specifically, each mouse aged 2 months was placed at the corner of an arm and allowed to freely move in the Y-shaped maze for 8 minutes. A value obtained by dividing the number of movements from the immediately preceding arm where the mouse stayed to an arm different from the immediately preceding arm where the mouse stayed by the number of movements in the arms was calculated as the rate of spontaneous alternation (score of alternation behavior; see Figure 14).

### [Preparation of human AD iPS cell]

Human iPS cells having KM670/671NL Sweden mutation (AD mutation) were commissioning-prepared by Applied StemCell, Inc. using CRISPR/Cas9 knockout system. Specifically, human normal iPS cells (ASE-9203, manufactured by Applied StemCell, Inc.) were transfected with a plasmid containing DNA encoding single-stranded guide (g) RNA (DNA consisting of the nucleotide sequence shown by SEQ ID NO: 17), a plasmid for expressing Cas9 gene fused with 2A peptide gene and GFP gene, and single-stranded donor DNA for introducing KM670/671NL Sweden mutation to human APP (single-stranded DNA consisting of the nucleotide sequence shown by SEQ ID NO: 18), by electroporation (1200 V, 30 ms, 1 pulse) using Neon transfection system (manufactured by Thermo Fisher Scientific Inc.). The cells were cultured for 24 to 48 hours in the presence of 0.4 µg/mL puromycin (selective drug). Then, the formed colonies (cell masses) were picked up, and a GFP-positive colony was transferred to a 96-well plate and cultured for 7 to 10 days. Human iPS cells having the KM670/671NL Sweden mutation of human APP heterozygously (AD/-) or homozygously (AD/AD) (referred to as "human AD/-iPS cells" and "human AD/AD iPS cells", respectively; hereinafter, these cells are collectively referred to as "human AD iPS cells") were confirmed according to the Sanger sequencing method by PCR using a primer set (forward primer consisting of the nucleotide sequence shown by SEQ ID NO: 19 and reverse primer consisting of the nucleotide sequence shown by SEQ ID NO: 20) .

### 2. Results

### [HMGB1 concentration in CSF sample derived from MCI patient is higher than that of control subject having no MCI]

The median value of HMGB1 concentrations in MCI patient-derived CSF samples was 1548 pg/mL, which was higher than the median value (229 pg/mL) of HMGB1 concentrations in non-MCI/AD control subject-derived CSF samples and the median value (327 pg/mL) of HMGB1 concentrations in AD patient-derived CSF samples (see Figure 1). Next, the non-MCI/AD control subjects were divided into two groups, healthy individuals and disease patients, which were then compared with the MCI patients. As a result, the median value (1548 pg/mL) of HMGB1 concentrations in the MCI patient-derived CSF samples was higher than the median value (338 pg/mL) of HMGB1 concentrations in healthy individual-derived CSF samples and the median value (133 pg/mL) of HMGB1 concentrations in disease patient-derived CSF samples (see Figure 3).

These results indicate that HMGB1 concentrations in MCI patient-derived biological samples are higher than those in biological samples derived from individuals (e.g., healthy individuals and dementia (e.g., AD) patients) other than MCI patients. In Figure 1, the interquartile ranges in the box plots of "cc", "MCI", and "AD" are 20 to 680.75 pg/mL, 205 to 2701 pg/mL, and 3 to 961.75 pg/mL, respectively. In Figure 3, the interquartile ranges in the box plots of "nc", "dc", "MCI", and "AD" are 56 to 796.5 pg/mL, 20 to 298 pg/mL, 205 to 2701 pg/mL, and 3 to 961.75 pg/mL, respectively.

Next, on the basis of the results of measuring the HMGB1 concentrations in the CSF samples derived from the non-MCI/AD control subjects (specifically, healthy individuals and disease patients), the MCI patients, and the AD patients, ROC curves were prepared in order to discriminate between the MCI patients and the non-MCI/AD control subjects or the AD patients (see Figures 2 and 4). Cutoff values were calculated. As a result, when 754 was set as a cutoff value for discriminating between the MCI patients and the non-MCI/AD control subjects, the sensitivity (ratio of test positive individuals to the MCI patients) and specificity (ratio of test negative individuals to the non-MCI/AD control subjects) of the MCI patients were 86% and 80%, respectively. When 754 was set as a cutoff value for discriminating between the MCI patients and the AD patients, the sensitivity (ratio of test positive individuals to the MCI patients) and specificity (ratio of test negative individuals to the AD patients) of the MCI patients were 86% and 70%, respectively. When 992 was set as a cutoff value for discriminating between the MCI patients and the healthy individuals, the sensitivity (ratio of test positive individuals to the MCI patients) and specificity (ratio of test negative individuals to the healthy individuals) of the MCI patients were 76% and 84%, respectively. When 754 was set as a cutoff value for discriminating between the MCI patients and the disease patients, the sensitivity (ratio of test positive individuals to the MCI patients) and specificity (ratio of test negative individuals to the disease patients) of the MCI patients were 86% and 91%, respectively.

These results indicate that whether or not test subjects have MCI can be accurately determined with HMGB1 concentrations as an index by measuring the HMGB1 concentrations in biological samples derived from the test subjects, and setting an appropriate cutoff value. AUC (area under the curve) in the ROC curve for discriminating between the MCI patients and the non-MCI/AD control subjects (see Figure 2A) is 0.887. AUC in the ROC curve for discriminating between the MCI patients and the AD patients (see Figure 2B) is 0.809. AUC in the ROC curve for discriminating between the MCI patients and the healthy individuals (see Figure 4A) is 0.861. AUC in the ROC curve for discriminating between the MCI patients and the disease patients (see Figure 4B) is 0.931. Therefore, it is evident that the ability to discriminate between MCI patients and other subjects is high.

### [AD mouse model aged around 6 months reflects condition of MCI]

HMGB1 is known as one of DAMPs released from necrotic cells (see the documents "Nature 418, 191-195 (2002)" and "Nat Rev Immunol 5, 331-342 (2005)"). Accordingly, the numbers of necrotic cell were measured in the brain neurons of three types of test subjects (non-MCI/AD disease patients, MCI patients, and AD patients). As a result, necrotic cells were rarely detected in the non-MCI/AD disease patients, whereas the increased numbers of necrotic cell were found in the MCI patients (see Figure 5C). On the other hand, the numbers of necrotic cell in the AD patients were rather lower than those in the MCI patients (see Figure 5C).

These results indicate that necrosis occurs in the brain neurons of MCI patients.

Further, the numbers of necrotic cell were measured in the brain neurons of two types of AD mouse models (5×FAD mice and APP-KI mice). As a result, in both the mice, the numbers of necrotic cell were increased from 1 to 6 months of age, whereas the numbers of necrotic cell were rather decreased after 6 months of age (see Figures 5A and 5B).

These results and the results about humans mentioned above, taken together into consideration, suggest that: AD mouse models aged around 6 months reflect the condition (symptoms) of MCI; AD mouse models before 6 months of age (specifically, AD mouse models aged 1 month) reflect the condition before MCI development or at a very early stage after MCI development; and AD mouse models after 6 months of age (specifically, AD mouse models aged 12 to 18 months) reflect the condition (symptoms) of AD patients.

### [Hippo pathway-dependent necrosis occurs due to MCI in brain neuron]

Next, in order to examine necrotic cells in brain neurons in more detail, ER and amyloid β in the neurons were analyzed by live imaging with ER-Tracker Red and BTA1, respectively, as indexes. As a result, no BTA1 (i.e., amyloid β) was detected and ER was normal in the brain neurons of control normal mice, whereas amyloid β was detected and ER dilation and instability were found in the brain neurons of 5×FAD mice aged 1 to 6 months (see Figures 6 and 7).

It has been reported that ER dilation and instability are related to TRIAD (transcriptional repression induced cell death), i.e., Hippo pathway-dependent necrosis (see the documents "J Cell Biol 172, 589-604 (2006)", "Cell Death Dis 7, e2207 (2016)", and "Hum Mol Genet 25, 4749-4770 (2016)"). Accordingly, the expression of YAP, a major factor of the Hippo pathway, was analyzed in brain neurons derived from the three types of test subjects. As a result, YAP was detected in the nuclei in the control non-MCI/AD disease patient-derived brain neurons, whereas the amounts of nuclear YAP were decreased in the AD patient-derived brain neurons and the amounts of nuclear YAP were further decreased in the MCI patient-derived brain neurons compared with the AD patient-derived brain neurons (see Figures 8 and 9A). It was also found that YAP was cytoplasmically colocalized with amyloid β in the brain neurons derived from AD patients and the MCI patients (see Figure 8A). It is known that LATS1 is activated by the phosphorylation of serine 909 (Ser909), thereby suppressing the nuclear translocation of YAP (see the document "Genes Dev 30, 1-17 (2016)"). Therefore, the levels of the phosphorylated LATS1 (pLATS1) were analyzed. As a result, the pLATS1 levels in the AD patient-derived brain neurons were higher than those in the non-MCI/AD disease patient-derived brain neurons, and the pLATS1 levels were further increased in the MCI patient-derived brain neurons compared with the AD patient-derived brain neurons (see Figure 9B). The decreased amounts of nuclear YAP were also found in two types of AD mouse models (5×FAD mice and APP-KI mice) aged 3 months (see Figures 9C and 9D).

These results indicate that Hippo pathway-dependent necrosis (TRIAD) occurred in onset of MCI in the human and mouse brain neurons.

### [Nuclear YAP-increasing substance or YAP has action of suppressing Hippo pathway-dependent necrosis caused by MCI in brain neuron]

S1P suppresses the phosphorylation of LATS1 via S1P receptor, thereby promoting the nuclear translocation of YAP. S1P as well as YAP and its isoforms are known to have an effect of suppressing Hippo pathway-dependent necrosis (see the documents "J Cell Biol 172, 589-604 (2006)", "Cell Death Dis 7, e2207 (2016)", and "Hum Mol Genet 25, 4749-4770 (2016)"). Accordingly, whether to be able to suppress Hippo pathway-dependent necrosis caused by MCI was tested using S1P and rat YAPΔC-ins61.

First, S1P or AAV-YAPΔC-ins61 was administered to 5×FAD mice at 1 month of age (i.e., before MCI development or at a very early stage after MCI development). As a result, it was confirmed that the amounts of nuclear YAP in brain neurons were increased and/or restored to the levels of normal mice, at 6 months of age (i.e., in onset of MCI) as compared with 5×FAD mice without administration thereof (see Figure 10). Next, the extracellular amyloid β levels of brain tissues were analyzed. As a result, in the 5×FAD mice given S1P or AAV-YAPΔC-ins61 at 1 month of age, the amyloid β levels were lower at 6 months of age than those in 5×FAD mice without administration thereof (see Figure 11). As a result of further analyzing ER in brain neurons, the administration of S1P or AAV-YAPΔCins61 to 5×FAD mice at 1 month of age significantly resolved ER dilation and instability at 6 months of age (see Figures 13 and 15) and restored ER to the state of normal mice (Figures 12 and 14).

These results indicate that a nuclear YAP-increasing substance such as S1P or YAP such as AAV-YAPΔC-ins61 has action of suppressing Hippo pathway-dependent necrosis caused by MCI in brain neurons.

### [Nuclear YAP-increasing substance or YAP has action of restoring cognitive function declined by MCI]

It is known that in brain neurons, HMGB1 is leaked out of cells that have necrosed, thereby phosphorylating serine 46 (Ser46) in MARCKS (pSer46-MARCKS) (see the document "SCIENTIFIC REPORT 25, 31895 (2016)"). Accordingly, the pSer46-MARCKS levels of brain neurons were analyzed. As a result, in the 5×FAD mice given S1P or AAV-YAPΔC-ins61 at 1 month of age, the pSer46-MARCKS levels were decreased to the levels of normal mice, at 6 months of age as compared with 5×FAD mice without administration thereof (see Figure 16).

These results indicate that S1P or AAV-YAPΔC-ins61 has action of suppressing increase in the number of necrotic cell.

As a result of further analyzing the cognitive functions of mice with scores of alternation behavior as indexes using a Y-shaped maze, it was first confirmed that 5×FAD mice were inferior in cognitive function at 6 months of age (i.e., in onset of MCI) to normal mice (see Figure 17). Next, it was found that the cognitive functions of 5×FAD mice given S1P or AAV-YAPΔC-ins61 at 1 month of age (i.e., before MCI development or at a very early stage after MCI development) were equivalent to the ones of normal mice, at 6 months of age as compared with 5×FAD mice without administration thereof (see Figures 17A and 17B) .

These results indicate that a nuclear YAP-increasing substance such as S1P or YAP such as AAV-YAPΔC-ins61 has action of preventing decline in cognitive function caused by MCI.

It was further found that the cognitive functions of 5×FAD mice given S1P or AAV-YAPΔC-ins61 at 5 months of age (i.e., in onset of MCI) were equivalent to the ones of normal mice, at 6 months of age as compared with 5×FAD mice without administration thereof (see Figures 17C and 17D) .

These results indicate that a nuclear YAP-increasing substance such as S1P or YAP such as AAV-YAPΔC-ins61 has action of restoring cognitive functions declined by MCI.

### Industrial Applicability

The present invention contributes to the prevention or treatment of mild cognitive impairment and the prevention of dementia (e.g., Alzheimer's dementia) to which mild cognitive impairment progresses.

## Claims

1. An agent for preventing or treating mild cognitive impairment, comprising mammalian YAP, a polynucleotide encoding mammalian YAP, or a substance capable of increasing an amount of mammalian YAP in a nucleus of a brain neuron.

2. The agent according to claim 1, wherein the mammalian YAP is a polypeptide comprising an amino acid sequence having at least 80% sequence identity to the amino acid sequence shown by SEQ ID NO: 1, and the substance capable of increasing an amount of mammalian YAP in a nucleus of a brain neuron is sphingosine 1-phosphate.

3. The agent according to claim 1 or 2, wherein the mild cognitive impairment is a condition having an elevated concentration of HMGB1 in cerebrospinal fluid as compared with a control subject without mild cognitive impairment.

4. The agent according to claim 3, wherein the control subject without mild cognitive impairment is a healthy individual or an Alzheimer's disease patient.


SEQUENCE LISTING

<110>  NATIONAL UNIVERSITY CORPORATION TOKYO MEDICAL AND DENTAL UNIVERSITY

<120>  Agent for preventing or treating Mild Cognitive Impairment

<130>  P19-010P

<150>  JP 2019-145259
<151>  2019-08-07

<160>  20

<170>  PatentIn version 3.1

<210>  1
<211>  366
<212>  PRT
<213>  Rattus norvegicus

<220>
<221>  MISC_FEATURE
<223>  Inventor:OKAZAWA, Hitoshi


<220>
<221>  MISC_FEATURE
<223>  Rat YAP delta C-ins61


<400>  1

Met Glu Pro Ala Gln Gln Pro Pro Pro Gln Pro Ala Pro Gln Gly Pro
1               5                   10                  15


Ala Pro Pro Ser Val Ser Pro Ala Gly Thr Pro Ala Ala Pro Pro Ala
            20                  25                  30


Pro Pro Ala Gly His Gln Val Val His Val Arg Gly Asp Ser Glu Thr
        35                  40                  45


Asp Leu Glu Ala Leu Phe Asn Ala Val Met Asn Pro Lys Thr Ala Asn
    50                  55                  60


Val Pro Gln Thr Val Pro Met Arg Leu Arg Lys Leu Pro Asp Ser Phe
65                  70                  75                  80


Phe Lys Pro Pro Glu Pro Lys Ser His Ser Arg Gln Ala Ser Thr Asp
                85                  90                  95


Ala Gly Thr Ala Gly Ala Leu Thr Pro Gln His Val Arg Ala His Ser
            100                 105                 110


Ser Pro Ala Ser Leu Gln Leu Gly Ala Gly Thr Leu Thr Ala Ser Gly
        115                 120                 125

Val Val Ser Gly Pro Ala Ala Thr Pro Ala Ala Gln His Leu Arg Gln
    130                 135                 140

Ser Ser Phe Glu Ile Pro Asp Asp Val Pro Leu Pro Ala Gly Trp Glu
145                 150                 155                 160

Met Ala Lys Thr Ser Ser Gly Gln Arg Tyr Phe Leu Asn His Asn Asp
                165                 170                 175

Gln Thr Thr Thr Trp Gln Asp Pro Arg Lys Ala Met Leu Ser Gln Leu
            180                 185                 190

Asn Val Pro Thr Ser Ala Ser Pro Ala Val Pro Gln Thr Leu Met Asn
        195                 200                 205

Ser Ala Ser Gly Pro Leu Pro Asp Gly Trp Glu Gln Ala Met Thr Gln
    210                 215                 220

Asp Gly Glu Val Tyr Tyr Ile Asn His Lys Asn Lys Thr Thr Ser Trp
225                 230                 235                 240

Leu Asp Pro Arg Leu Asp Pro Arg Phe Ala Met Asn Gln Arg Ile Thr
                245                 250                 255

Gln Ser Ala Pro Val Lys Gln Pro Pro Pro Leu Ala Pro Gln Ser Pro
            260                 265                 270

Gln Gly Gly Val Leu Gly Gly Gly Ser Ser Asn Gln Gln Gln Gln Ile
        275                 280                 285

Gln Leu Gln Gln Leu Gln Met Glu Lys Glu Arg Leu Arg Leu Lys Gln
    290                 295                 300

Gln Glu Leu Phe Arg Gln Ala Ile Arg Asn Ile Asn Pro Ser Thr Ala
305                 310                 315                 320

Asn Ala Pro Lys Cys Gln Thr Val Arg Ala Gly Ile Ser Ser Pro Gln
                325                 330                 335

Pro Val Ala Leu Thr Gly Ala Gly Trp Arg Asp Ser Glu Cys Ser Val
            340                 345                 350

Phe Ser Arg Asp Asp Ser Gly Ile Glu Asp Asn Asp Asn Gln
        355                 360                 365

<210>  2
<211>  1101

<212>  DNA
<213>  Rattus norvegicus

<220>
<221>  misc_feature
<223>  cDNA encoding rat YAP delta C-ins61


<400>  2
atggagcccg cgcaacagcc gccgccccag ccggccccgc aaggccccgc gccgccgtcg      60

gtgtctccgg ccgggacccc cgcggccccg cccgcacccc cggcgggtca ccaggtcgtg     120

cacgtccgcg gggactcgga gaccgacctg gaagcgcttt tcaacgccgt catgaacccc     180

aagacggcca acgtgccaca gaccgtgccc atgaggcttc gcaagctgcc cgactccttc     240

ttcaagccgc ctgagcccaa gtcccactcg cgacaggcca gtaccgatgc gggcactgct     300

ggagccctga ctccccagca cgttcgagct cactcgtctc cagcctccct gcagctgggg     360

gccgggacac tcacggccag tggtgttgtc tctggcccgg ccgccacccc tgctgctcaa     420

catctcagac agtcttcctt tgagatccct gatgatgtac cattgccagc aggctgggag     480

atggccaaga cctcttctgg tcagagatac ttcttaaatc acaatgatca gacaacaaca     540

tggcaggacc cccggaaggc catgctctcc caactgaacg ttcctacatc tgccagccca     600

gcagtgcccc agacgctgat gaactctgcc tcagggcctc ttcctgatgg atgggagcaa     660

gccatgactc aggatggaga agtttactac ataaaccata agaacaagac cacatcctgg     720

ctggacccaa ggcttgaccc tcgttttgcc atgaaccaga ggatcactca gagtgctcca     780

gtgaagcagc ccccaccctt ggctccccag agcccacagg gaggcgtcct gggtggaggc     840

agctcaaacc agcagcagca gatacagctg cagcagctac agatggagaa ggagaggctg     900

cgattgaaac agcaggagtt atttcggcag gcaatacgga atatcaatcc cagcacagca     960

aatgctccaa aatgtcagac cgtcagagcg ggaattagct ctccgcagcc agttgccctc    1020

actggagcag gatggaggga ctcagaatgc agtgtcttct cccgggatga ctcaggaatt    1080

gaggacaatg acaaccaata g                                              1101


<210>  3
<211>  350
<212>  PRT
<213>  Rattus norvegicus

<220>
<221>  MISC_FEATURE
<223>  Rat YAP delta C-ins13


<400>  3
   Met Glu Pro Ala Gln Gln Pro Pro Pro Gln Pro Ala Pro Gln Gly Pro
   1               5                   10                  15
   Ala Pro Pro Ser Val Ser Pro Ala Gly Thr Pro Ala Ala Pro Pro Ala
               20                  25                  30
   Pro Pro Ala Gly His Gln Val Val His Val Arg Gly Asp Ser Glu Thr
           35                  40                  45
   Asp Leu Glu Ala Leu Phe Asn Ala Val Met Asn Pro Lys Thr Ala Asn
       50                  55                  60
   Val Pro Gln Thr Val Pro Met Arg Leu Arg Lys Leu Pro Asp Ser Phe
   65                  70                  75                  80
   Phe Lys Pro Pro Glu Pro Lys Ser His Ser Arg Gln Ala Ser Thr Asp
                   85                  90                  95
   Ala Gly Thr Ala Gly Ala Leu Thr Pro Gln His Val Arg Ala His Ser
               100                 105                 110
   Ser Pro Ala Ser Leu Gln Leu Gly Ala Gly Thr Leu Thr Ala Ser Gly
           115                 120                 125
   Val Val Ser Gly Pro Ala Ala Thr Pro Ala Ala Gln His Leu Arg Gln
       130                 135                 140
   Ser Ser Phe Glu Ile Pro Asp Asp Val Pro Leu Pro Ala Gly Trp Glu
   145                 150                 155                 160
   Met Ala Lys Thr Ser Ser Gly Gln Arg Tyr Phe Leu Asn His Asn Asp
                   165                 170                 175
   Gln Thr Thr Thr Trp Gln Asp Pro Arg Lys Ala Met Leu Ser Gln Leu
               180                 185                 190
   Asn Val Pro Thr Ser Ala Ser Pro Ala Val Pro Gln Thr Leu Met Asn
           195                 200                 205
   Ser Ala Ser Gly Pro Leu Pro Asp Gly Trp Glu Gln Ala Met Thr Gln
       210                 215                 220
   Asp Gly Glu Val Tyr Tyr Ile Asn His Lys Asn Lys Thr Thr Ser Trp
   225                 230                 235                 240
   Leu Asp Pro Arg Leu Asp Pro Arg Phe Ala Met Asn Gln Arg Ile Thr
                   245                 250                 255
   Gln Ser Ala Pro Val Lys Gln Pro Pro Pro Leu Ala Pro Gln Ser Pro
               260                 265                 270
   Gln Gly Gly Val Leu Gly Gly Gly Ser Ser Asn Gln Gln Gln Gln Ile
           275                 280                 285
   Gln Leu Gln Gln Leu Gln Met Glu Lys Glu Arg Leu Arg Leu Lys Gln
       290                 295                 300
   Gln Glu Leu Phe Arg Gln Thr Val Arg Ala Gly Ile Ser Ser Pro Gln
   305                 310                 315                 320
   Pro Val Ala Leu Thr Gly Ala Gly Trp Arg Asp Ser Glu Cys Ser Val
                   325                 330                 335
   Phe Ser Arg Asp Asp Ser Gly Ile Glu Asp Asn Asp Asn Gln
               340                 345                 350

<210>  4
<211>  354
<212>  PRT
<213>  Rattus norvegicus

<220>
<221>  MISC_FEATURE
<223>  Rat YAP delta C-ins25

<400>  4

Met Glu Pro Ala Gln Gln Pro Pro Pro Gln Pro Ala Pro Gln Gly Pro
1               5                   10                  15

Ala Pro Pro Ser Val Ser Pro Ala Gly Thr Pro Ala Ala Pro Pro Ala
            20                  25                  30

Pro Pro Ala Gly His Gln Val Val His Val Arg Gly Asp Ser Glu Thr
        35                  40                  45

Asp Leu Glu Ala Leu Phe Asn Ala Val Met Asn Pro Lys Thr Ala Asn
    50                  55                  60

Val Pro Gln Thr Val Pro Met Arg Leu Arg Lys Leu Pro Asp Ser Phe
65                  70                  75                  80

Phe Lys Pro Pro Glu Pro Lys Ser His Ser Arg Gln Ala Ser Thr Asp
                85                  90                  95

Ala Gly Thr Ala Gly Ala Leu Thr Pro Gln His Val Arg Ala His Ser
            100                 105                 110

Ser Pro Ala Ser Leu Gln Leu Gly Ala Gly Thr Leu Thr Ala Ser Gly

115                 120                 125

Val Val Ser Gly Pro Ala Ala Thr Pro Ala Ala Gln His Leu Arg Gln
    130                 135                 140

Ser Ser Phe Glu Ile Pro Asp Asp Val Pro Leu Pro Ala Gly Trp Glu
145                 150                 155                 160

Met Ala Lys Thr Ser Ser Gly Gln Arg Tyr Phe Leu Asn His Asn Asp
                165                 170                 175

Gln Thr Thr Thr Trp Gln Asp Pro Arg Lys Ala Met Leu Ser Gln Leu
            180                 185                 190

Asn Val Pro Thr Ser Ala Ser Pro Ala Val Pro Gln Thr Leu Met Asn
        195                 200                 205

Ser Ala Ser Gly Pro Leu Pro Asp Gly Trp Glu Gln Ala Met Thr Gln
    210                 215                 220

Asp Gly Glu Val Tyr Tyr Ile Asn His Lys Asn Lys Thr Thr Ser Trp
225                 230                 235                 240

Leu Asp Pro Arg Leu Asp Pro Arg Phe Ala Met Asn Gln Arg Ile Thr
                245                 250                 255

Gln Ser Ala Pro Val Lys Gln Pro Pro Pro Leu Ala Pro Gln Ser Pro
            260                 265                 270

Gln Gly Gly Val Leu Gly Gly Gly Ser Ser Asn Gln Gln Gln Gln Ile
        275                 280                 285

Gln Leu Gln Gln Leu Gln Met Glu Lys Glu Arg Leu Arg Leu Lys Gln
    290                 295                 300

Gln Glu Leu Phe Arg Gln Val Arg Pro Gln Thr Val Arg Ala Gly Ile
305                 310                 315                 320

Ser Ser Pro Gln Pro Val Ala Leu Thr Gly Ala Gly Trp Arg Asp Ser
                325                 330                 335

Glu Cys Ser Val Phe Ser Arg Asp Asp Ser Gly Ile Glu Asp Asn Asp
            340                 345                 350

Asn Gln

<210>  5
<211>  469
<212>  PRT
<213>  Rattus norvegicus

<220>
<221>  MISC_FEATURE
<223>  Rat YAP


<400>  5

Met Glu Pro Ala Gln Gln Pro Pro Pro Gln Pro Ala Pro Gln Gly Pro
1               5                   10                  15

Ala Pro Pro Ser Val Ser Pro Ala Gly Thr Pro Ala Ala Pro Pro Ala
            20                  25                  30

Pro Pro Ala Gly His Gln Val Val His Val Arg Gly Asp Ser Glu Thr
        35                  40                  45

Asp Leu Glu Ala Leu Phe Asn Ala Val Met Asn Pro Lys Thr Ala Asn
    50                  55                  60

Val Pro Gln Thr Val Pro Met Arg Leu Arg Lys Leu Pro Asp Ser Phe
65                  70                  75                  80

Phe Lys Pro Pro Glu Pro Lys Ser His Ser Arg Gln Ala Ser Thr Asp
                85                  90                  95

Ala Gly Thr Ala Gly Ala Leu Thr Pro Gln His Val Arg Ala His Ser
            100                 105                 110

Ser Pro Ala Ser Leu Gln Leu Gly Ala Gly Thr Leu Thr Ala Ser Gly
        115                 120                 125

Val Val Ser Gly Pro Ala Ala Thr Pro Ala Ala Gln His Leu Arg Gln
    130                 135                 140

Ser Ser Phe Glu Ile Pro Asp Asp Val Pro Leu Pro Ala Gly Trp Glu
145                 150                 155                 160

Met Ala Lys Thr Ser Ser Gly Gln Arg Tyr Phe Leu Asn His Asn Asp
                165                 170                 175

Gln Thr Thr Thr Trp Gln Asp Pro Arg Lys Ala Met Leu Ser Gln Leu
            180                 185                 190

Asn Val Pro Thr Ser Ala Ser Pro Ala Val Pro Gln Thr Leu Met Asn
        195                 200                 205

Ser Ala Ser Gly Pro Leu Pro Asp Gly Trp Glu Gln Ala Met Thr Gln
    210                 215                 220

Asp Gly Glu Val Tyr Tyr Ile Asn His Lys Asn Lys Thr Thr Ser Trp
225                 230                 235                 240

Leu Asp Pro Arg Leu Asp Pro Arg Phe Ala Met Asn Gln Arg Ile Thr
                245                 250                 255

Gln Ser Ala Pro Val Lys Gln Pro Pro Pro Leu Ala Pro Gln Ser Pro
            260                 265                 270

Gln Gly Gly Val Leu Gly Gly Gly Ser Ser Asn Gln Gln Gln Gln Ile
        275                 280                 285

Gln Leu Gln Gln Leu Gln Met Glu Lys Glu Arg Leu Arg Leu Lys Gln
    290                 295                 300

Gln Glu Leu Phe Arg Gln Glu Leu Ala Leu Arg Ser Gln Leu Pro Ser
305                 310                 315                 320

Leu Glu Gln Asp Gly Gly Thr Gln Asn Ala Val Ser Ser Pro Gly Met
                325                 330                 335

Thr Gln Glu Leu Arg Thr Met Thr Thr Asn Ser Ser Asp Pro Phe Leu
            340                 345                 350

Asn Ser Gly Thr Tyr His Ser Arg Asp Glu Ser Thr Asp Ser Gly Leu
        355                 360                 365

Ser Met Ser Ser Tyr Ser Ile Pro Arg Thr Pro Asp Asp Phe Leu Asn
    370                 375                 380

Ser Val Asp Glu Met Asp Thr Gly Asp Thr Ile Ser Gln Ser Thr Leu
385                 390                 395                 400

Pro Ser Gln Gln Ser Arg Phe Pro Asp Tyr Leu Glu Ala Leu Pro Gly
                405                 410                 415

Thr Asn Val Asp Leu Gly Thr Leu Glu Gly Asp Ala Met Asn Ile Glu
            420                 425                 430

Gly Glu Glu Leu Met Pro Ser Leu Gln Glu Ala Leu Ser Ser Glu Ile
        435                 440                 445

Leu Asp Val Glu Ser Val Leu Ala Ala Thr Lys Leu Asp Lys Glu Ser
    450                 455                 460

Phe Leu Thr Trp Leu
465

<210> 6
<211> 472
<212> PRT
<213> Mus musculus

<220>
<221> MISC_FEATURE
<223> Mouse YAP

<400> 6

Met Glu Pro Ala Gln Gln Pro Pro Pro Gln Pro Ala Pro Gln Gly Pro
1               5                   10                  15

Ala Pro Pro Ser Val Ser Pro Ala Gly Thr Pro Ala Ala Pro Pro Ala
            20                  25                  30

Pro Pro Ala Gly His Gln Val Val His Val Arg Gly Asp Ser Glu Thr
        35                  40                  45

Asp Leu Glu Ala Leu Phe Asn Ala Val Met Asn Pro Lys Thr Ala Asn
    50                  55                  60

Val Pro Gln Thr Val Pro Met Arg Leu Arg Lys Leu Pro Asp Ser Phe
65                  70                  75                  80

Phe Lys Pro Pro Glu Pro Lys Ser His Ser Arg Gln Ala Ser Thr Asp
                85                  90                  95

Ala Gly Thr Ala Gly Ala Leu Thr Pro Gln His Val Arg Ala His Ser
            100                 105                 110

Ser Pro Ala Ser Leu Gln Leu Gly Ala Val Ser Pro Gly Thr Leu Thr
        115                 120                 125

Ala Ser Gly Val Val Ser Gly Pro Ala Ala Ala Pro Ala Ala Gln His
    130                 135                 140

Leu Arg Gln Ser Ser Phe Glu Ile Pro Asp Asp Val Pro Leu Pro Ala
145                 150                 155                 160

Gly Trp Glu Met Ala Lys Thr Ser Ser Gly Gln Arg Tyr Phe Leu Asn
                165                 170                 175

His Asn Asp Gln Thr Thr Thr Trp Gln Asp Pro Arg Lys Ala Met Leu

180                 185                 190

Ser Gln Leu Asn Val Pro Ala Pro Ala Ser Pro Ala Val Pro Gln Thr
        195                 200                 205

Leu Met Asn Ser Ala Ser Gly Pro Leu Pro Asp Gly Trp Glu Gln Ala
    210                 215                 220

Met Thr Gln Asp Gly Glu Val Tyr Tyr Ile Asn His Lys Asn Lys Thr
225                 230                 235                 240

Thr Ser Trp Leu Asp Pro Arg Leu Asp Pro Arg Phe Ala Met Asn Gln
                245                 250                 255

Arg Ile Thr Gln Ser Ala Pro Val Lys Gln Pro Pro Pro Leu Ala Pro
            260                 265                 270

Gln Ser Pro Gln Gly Gly Val Leu Gly Gly Gly Ser Ser Asn Gln Gln
        275                 280                 285

Gln Gln Ile Gln Leu Gln Gln Leu Gln Met Glu Lys Glu Arg Leu Arg
    290                 295                 300

Leu Lys Gln Gln Glu Leu Phe Arg Gln Glu Leu Ala Leu Arg Ser Gln
305                 310                 315                 320

Leu Pro Thr Leu Glu Gln Asp Gly Gly Thr Pro Asn Ala Val Ser Ser
                325                 330                 335

Pro Gly Met Ser Gln Glu Leu Arg Thr Met Thr Thr Asn Ser Ser Asp
            340                 345                 350

Pro Phe Leu Asn Ser Gly Thr Tyr His Ser Arg Asp Glu Ser Thr Asp
        355                 360                 365

Ser Gly Leu Ser Met Ser Ser Tyr Ser Ile Pro Arg Thr Pro Asp Asp
    370                 375                 380

Phe Leu Asn Ser Val Asp Glu Met Asp Thr Gly Asp Thr Ile Ser Gln
385                 390                 395                 400

Ser Thr Leu Pro Ser Gln Gln Ser Arg Phe Pro Asp Tyr Leu Glu Ala
                405                 410                 415

Leu Pro Gly Thr Asn Val Asp Leu Gly Thr Leu Glu Gly Asp Ala Met
            420                 425                 430

Asn Ile Glu Gly Glu Glu Leu Met Pro Ser Leu Gln Glu Ala Leu Ser
        435                 440                 445

Ser Glu Ile Leu Asp Val Glu Ser Val Leu Ala Ala Thr Lys Leu Asp
    450                 455                 460

Lys Glu Ser Phe Leu Thr Trp Leu
465                 470

<210>  7
<211>  488
<212>  PRT
<213>  Homo sapiens

<220>
<221>  MISC_FEATURE
<223>  Human YAP2

<400>  7

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly
            20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ser Ser
        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Gly Pro Leu Pro Asp Gly Trp Glu Gln Ala Met
225                 230                 235                 240

Thr Gln Asp Gly Glu Ile Tyr Tyr Ile Asn His Lys Asn Lys Thr Thr
                245                 250                 255

Ser Trp Leu Asp Pro Arg Leu Asp Pro Arg Phe Ala Met Asn Gln Arg
            260                 265                 270

Ile Ser Gln Ser Ala Pro Val Lys Gln Pro Pro Pro Leu Ala Pro Gln
        275                 280                 285

Ser Pro Gln Gly Gly Val Met Gly Gly Ser Asn Ser Asn Gln Gln Gln
    290                 295                 300

Gln Met Arg Leu Gln Gln Leu Gln Met Glu Lys Glu Arg Leu Arg Leu
305                 310                 315                 320

Lys Gln Gln Glu Leu Leu Arg Gln Glu Leu Ala Leu Arg Ser Gln Leu
                325                 330                 335

Pro Thr Leu Glu Gln Asp Gly Gly Thr Gln Asn Pro Val Ser Ser Pro
            340                 345                 350

Gly Met Ser Gln Glu Leu Arg Thr Met Thr Thr Asn Ser Ser Asp Pro
        355                 360                 365

Phe Leu Asn Ser Gly Thr Tyr His Ser Arg Asp Glu Ser Thr Asp Ser
    370                 375                 380

Gly Leu Ser Met Ser Ser Tyr Ser Val Pro Arg Thr Pro Asp Asp Phe
385                 390                 395                 400

Leu Asn Ser Val Asp Glu Met Asp Thr Gly Asp Thr Ile Asn Gln Ser
                405                 410                 415

Thr Leu Pro Ser Gln Gln Asn Arg Phe Pro Asp Tyr Leu Glu Ala Ile
            420                 425                 430

Pro Gly Thr Asn Val Asp Leu Gly Thr Leu Glu Gly Asp Gly Met Asn
        435                 440                 445

Ile Glu Gly Glu Glu Leu Met Pro Ser Leu Gln Glu Ala Leu Ser Ser
    450                 455                 460

Asp Ile Leu Asn Asp Met Glu Ser Val Leu Ala Ala Thr Lys Leu Asp
465                 470                 475                 480

Lys Glu Ser Phe Leu Thr Trp Leu
                485

<210> 8
<211> 508
<212> PRT
<213> Homo sapiens

<220>
<221> MISC_FEATURE
<223> Human YAP1-2 delta

<400> 8

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly
            20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ser Ser

        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Gly Pro Leu Pro Asp Gly Trp Glu Gln Ala Met
225                 230                 235                 240

Thr Gln Asp Gly Glu Ile Tyr Tyr Ile Asn His Lys Asn Lys Thr Thr
                245                 250                 255

Ser Trp Leu Asp Pro Arg Leu Asp Pro Arg Phe Ala Met Asn Gln Arg
            260                 265                 270

Ile Ser Gln Ser Ala Pro Val Lys Gln Pro Pro Pro Leu Ala Pro Gln
        275                 280                 285

Ser Pro Gln Gly Gly Val Met Gly Gly Ser Asn Ser Asn Gln Gln Gln
    290                 295                 300

Gln Met Arg Leu Gln Gln Leu Gln Met Glu Lys Glu Arg Leu Arg Leu
305                 310                 315                 320

Lys Gln Gln Glu Leu Leu Arg Gln Val Arg Pro Gln Ala Met Arg Asn
                325                 330                 335

Ile Asn Pro Ser Thr Ala Asn Ser Pro Lys Cys Gln Glu Leu Ala Leu
            340                 345                 350

Arg Ser Gln Leu Pro Thr Leu Glu Gln Asp Gly Gly Thr Gln Asn Pro
        355                 360                 365

Val Ser Ser Pro Gly Met Ser Gln Glu Leu Arg Thr Met Thr Thr Asn
    370                 375                 380

Ser Ser Asp Pro Phe Leu Asn Ser Gly Thr Tyr His Ser Arg Asp Glu
385                 390                 395                 400

Ser Thr Asp Ser Gly Leu Ser Met Ser Ser Tyr Ser Val Pro Arg Thr
                405                 410                 415

Pro Asp Asp Phe Leu Asn Ser Val Asp Glu Met Asp Thr Gly Asp Thr
            420                 425                 430

Ile Asn Gln Ser Thr Leu Pro Ser Gln Gln Asn Arg Phe Pro Asp Tyr
        435                 440                 445

Leu Glu Ala Ile Pro Gly Thr Asn Val Asp Leu Gly Thr Leu Glu Gly
    450                 455                 460

Asp Gly Met Asn Ile Glu Gly Glu Glu Leu Met Pro Ser Leu Gln Glu
465                 470                 475                 480

Ala Leu Ser Ser Asp Ile Leu Asn Asp Met Glu Ser Val Leu Ala Ala
                485                 490                 495

Thr Lys Leu Asp Lys Glu Ser Phe Leu Thr Trp Leu
            500                 505


<210>  9
<211>  488
<212>  PRT
<213>  Homo sapiens

<220>
<221>  MISC_FEATURE
<223>  Human YAP1-2 alpha


<400>  9

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly
            20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ser Ser
        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Gly Pro Leu Pro Asp Gly Trp Glu Gln Ala Met
225                 230                 235                 240

Thr Gln Asp Gly Glu Ile Tyr Tyr Ile Asn His Lys Asn Lys Thr Thr
                245                 250                 255

Ser Trp Leu Asp Pro Arg Leu Asp Pro Arg Phe Ala Met Asn Gln Arg
            260                 265                 270

Ile Ser Gln Ser Ala Pro Val Lys Gln Pro Pro Pro Leu Ala Pro Gln
        275                 280                 285

Ser Pro Gln Gly Gly Val Met Gly Gly Ser Asn Ser Asn Gln Gln Gln
    290                 295                 300

Gln Met Arg Leu Gln Gln Leu Gln Met Glu Lys Glu Arg Leu Arg Leu
305                 310                 315                 320

Lys Gln Gln Glu Leu Leu Arg Gln Glu Leu Ala Leu Arg Ser Gln Leu
                325                 330                 335

Pro Thr Leu Glu Gln Asp Gly Gly Thr Gln Asn Pro Val Ser Ser Pro
            340                 345                 350

Gly Met Ser Gln Glu Leu Arg Thr Met Thr Thr Asn Ser Ser Asp Pro
        355                 360                 365

Phe Leu Asn Ser Gly Thr Tyr His Ser Arg Asp Glu Ser Thr Asp Ser
    370                 375                 380

Gly Leu Ser Met Ser Ser Tyr Ser Val Pro Arg Thr Pro Asp Asp Phe
385                 390                 395                 400

Leu Asn Ser Val Asp Glu Met Asp Thr Gly Asp Thr Ile Asn Gln Ser
                405                 410                 415

Thr Leu Pro Ser Gln Gln Asn Arg Phe Pro Asp Tyr Leu Glu Ala Ile
            420                 425                 430

Pro Gly Thr Asn Val Asp Leu Gly Thr Leu Glu Gly Asp Gly Met Asn
        435                 440                 445

Ile Glu Gly Glu Glu Leu Met Pro Ser Leu Gln Glu Ala Leu Ser Ser
    450                 455                 460

Asp Ile Leu Asn Asp Met Glu Ser Val Leu Ala Ala Thr Lys Leu Asp
465                 470                 475                 480

Lys Glu Ser Phe Leu Thr Trp Leu
                485

<210>  10
<211>  492
<212>  PRT
<213>  Homo sapiens

<220>
<221>  MISC_FEATURE
<223>  Human YAP1-2 beta

<400>  10

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly

20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ser Ser
        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Gly Pro Leu Pro Asp Gly Trp Glu Gln Ala Met
225                 230                 235                 240

Thr Gln Asp Gly Glu Ile Tyr Tyr Ile Asn His Lys Asn Lys Thr Thr
                245                 250                 255

Ser Trp Leu Asp Pro Arg Leu Asp Pro Arg Phe Ala Met Asn Gln Arg
            260                 265                 270

Ile Ser Gln Ser Ala Pro Val Lys Gln Pro Pro Pro Leu Ala Pro Gln
        275                 280                 285

Ser Pro Gln Gly Gly Val Met Gly Gly Ser Asn Ser Asn Gln Gln Gln
    290                 295                 300

Gln Met Arg Leu Gln Gln Leu Gln Met Glu Lys Glu Arg Leu Arg Leu
305                 310                 315                 320

Lys Gln Gln Glu Leu Leu Arg Gln Val Arg Pro Gln Glu Leu Ala Leu
                325                 330                 335

Arg Ser Gln Leu Pro Thr Leu Glu Gln Asp Gly Gly Thr Gln Asn Pro
            340                 345                 350

Val Ser Ser Pro Gly Met Ser Gln Glu Leu Arg Thr Met Thr Thr Asn
        355                 360                 365

Ser Ser Asp Pro Phe Leu Asn Ser Gly Thr Tyr His Ser Arg Asp Glu
    370                 375                 380

Ser Thr Asp Ser Gly Leu Ser Met Ser Ser Tyr Ser Val Pro Arg Thr
385                 390                 395                 400

Pro Asp Asp Phe Leu Asn Ser Val Asp Glu Met Asp Thr Gly Asp Thr
                405                 410                 415

Ile Asn Gln Ser Thr Leu Pro Ser Gln Gln Asn Arg Phe Pro Asp Tyr
            420                 425                 430

Leu Glu Ala Ile Pro Gly Thr Asn Val Asp Leu Gly Thr Leu Glu Gly
        435                 440                 445

Asp Gly Met Asn Ile Glu Gly Glu Glu Leu Met Pro Ser Leu Gln Glu
    450                 455                 460

Ala Leu Ser Ser Asp Ile Leu Asn Asp Met Glu Ser Val Leu Ala Ala
465                 470                 475                 480

Thr Lys Leu Asp Lys Glu Ser Phe Leu Thr Trp Leu
                485                 490

<210>  11
<211>  504
<212>  PRT
<213>  Homo sapiens

<220>
<221>  MISC_FEATURE

<223>  Human YAP1-2 gamma

<400>  11

<210>  12
<211>  450
<212>  PRT
<213>  Homo sapiens

<220>
<221>  MISC_FEATURE
<223>  Human YAP1

<400>  12

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly
            20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ser Ser
        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Ala Met Asn Gln Arg Ile Ser Gln Ser Ala Pro
225                 230                 235                 240

Val Lys Gln Pro Pro Pro Leu Ala Pro Gln Ser Pro Gln Gly Gly Val
                245                 250                 255

Met Gly Gly Ser Asn Ser Asn Gln Gln Gln Gln Met Arg Leu Gln Gln
            260                 265                 270

Leu Gln Met Glu Lys Glu Arg Leu Arg Leu Lys Gln Gln Glu Leu Leu
        275                 280                 285

Arg Gln Glu Leu Ala Leu Arg Ser Gln Leu Pro Thr Leu Glu Gln Asp
    290                 295                 300

Gly Gly Thr Gln Asn Pro Val Ser Ser Pro Gly Met Ser Gln Glu Leu
305                 310                 315                 320

Arg Thr Met Thr Thr Asn Ser Ser Asp Pro Phe Leu Asn Ser Gly Thr
                325                 330                 335

Tyr His Ser Arg Asp Glu Ser Thr Asp Ser Gly Leu Ser Met Ser Ser
            340                 345                 350

Tyr Ser Val Pro Arg Thr Pro Asp Asp Phe Leu Asn Ser Val Asp Glu
        355                 360                 365

Met Asp Thr Gly Asp Thr Ile Asn Gln Ser Thr Leu Pro Ser Gln Gln
    370                 375                 380

Asn Arg Phe Pro Asp Tyr Leu Glu Ala Ile Pro Gly Thr Asn Val Asp
385                 390                 395                 400

Leu Gly Thr Leu Glu Gly Asp Gly Met Asn Ile Glu Gly Glu Glu Leu
                405                 410                 415

Met Pro Ser Leu Gln Glu Ala Leu Ser Ser Asp Ile Leu Asn Asp Met
            420                 425                 430

Glu Ser Val Leu Ala Ala Thr Lys Leu Asp Lys Glu Ser Phe Leu Thr
        435                 440                 445

Trp Leu
    450

<210>  13
<211>  466
<212>  PRT
<213>  Homo sapiens

<220>
<221>  MISC_FEATURE
<223>  Human YAP1-1 gamma

<400>  13

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly
            20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ser Ser
        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Ala Met Asn Gln Arg Ile Ser Gln Ser Ala Pro
225                 230                 235                 240

Val Lys Gln Pro Pro Pro Leu Ala Pro Gln Ser Pro Gln Gly Gly Val
                245                 250                 255

Met Gly Gly Ser Asn Ser Asn Gln Gln Gln Gln Met Arg Leu Gln Gln
            260                 265                 270

Leu Gln Met Glu Lys Glu Arg Leu Arg Leu Lys Gln Gln Glu Leu Leu
        275                 280                 285

Arg Gln Ala Met Arg Asn Ile Asn Pro Ser Thr Ala Asn Ser Pro Lys
    290                 295                 300

Cys Gln Glu Leu Ala Leu Arg Ser Gln Leu Pro Thr Leu Glu Gln Asp
305                 310                 315                 320

Gly Gly Thr Gln Asn Pro Val Ser Ser Pro Gly Met Ser Gln Glu Leu
                325                 330                 335

Arg Thr Met Thr Thr Asn Ser Ser Asp Pro Phe Leu Asn Ser Gly Thr
            340                 345                 350

Tyr His Ser Arg Asp Glu Ser Thr Asp Ser Gly Leu Ser Met Ser Ser
        355                 360                 365

Tyr Ser Val Pro Arg Thr Pro Asp Asp Phe Leu Asn Ser Val Asp Glu
    370                 375                 380

Met Asp Thr Gly Asp Thr Ile Asn Gln Ser Thr Leu Pro Ser Gln Gln
385                 390                 395                 400

Asn Arg Phe Pro Asp Tyr Leu Glu Ala Ile Pro Gly Thr Asn Val Asp
                405                 410                 415

Leu Gly Thr Leu Glu Gly Asp Gly Met Asn Ile Glu Gly Glu Glu Leu

420                 425                 430

Met Pro Ser Leu Gln Glu Ala Leu Ser Ser Asp Ile Leu Asn Asp Met
        435                 440                 445

Glu Ser Val Leu Ala Ala Thr Lys Leu Asp Lys Glu Ser Phe Leu Thr
    450                 455                 460

Trp Leu
465

<210>  14
<211>  470
<212>  PRT
<213>  Homo sapiens

<220>
<221>  MISC_FEATURE
<223>  Human YAP1-1 delta

<400>  14

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly
            20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ser Ser
        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Ala Met Asn Gln Arg Ile Ser Gln Ser Ala Pro
225                 230                 235                 240

Val Lys Gln Pro Pro Pro Leu Ala Pro Gln Ser Pro Gln Gly Gly Val
                245                 250                 255

Met Gly Gly Ser Asn Ser Asn Gln Gln Gln Gln Met Arg Leu Gln Gln
            260                 265                 270

Leu Gln Met Glu Lys Glu Arg Leu Arg Leu Lys Gln Gln Glu Leu Leu
        275                 280                 285

Arg Gln Val Arg Pro Gln Ala Met Arg Asn Ile Asn Pro Ser Thr Ala
    290                 295                 300

Asn Ser Pro Lys Cys Gln Glu Leu Ala Leu Arg Ser Gln Leu Pro Thr
305                 310                 315                 320

Leu Glu Gln Asp Gly Gly Thr Gln Asn Pro Val Ser Ser Pro Gly Met
                325                 330                 335

Ser Gln Glu Leu Arg Thr Met Thr Thr Asn Ser Ser Asp Pro Phe Leu
            340                 345                 350

Asn Ser Gly Thr Tyr His Ser Arg Asp Glu Ser Thr Asp Ser Gly Leu
        355                 360                 365

Ser Met Ser Ser Tyr Ser Val Pro Arg Thr Pro Asp Asp Phe Leu Asn
    370                 375                 380

Ser Val Asp Glu Met Asp Thr Gly Asp Thr Ile Asn Gln Ser Thr Leu
385                 390                 395                 400

Pro Ser Gln Gln Asn Arg Phe Pro Asp Tyr Leu Glu Ala Ile Pro Gly
                405                 410                 415

Thr Asn Val Asp Leu Gly Thr Leu Glu Gly Asp Gly Met Asn Ile Glu
            420                 425                 430

Gly Glu Glu Leu Met Pro Ser Leu Gln Glu Ala Leu Ser Ser Asp Ile
        435                 440                 445

Leu Asn Asp Met Glu Ser Val Leu Ala Ala Thr Lys Leu Asp Lys Glu
    450                 455                 460

Ser Phe Leu Thr Trp Leu
465                 470

<210>  15
<211>  450
<212>  PRT
<213>  Homo sapiens

<220>
<221>  MISC_FEATURE
<223>  Human YAP1-1 alpha

<400>  15

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly
            20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ser Ser
        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Ala Met Asn Gln Arg Ile Ser Gln Ser Ala Pro
225                 230                 235                 240

Val Lys Gln Pro Pro Pro Leu Ala Pro Gln Ser Pro Gln Gly Gly Val
                245                 250                 255

Met Gly Gly Ser Asn Ser Asn Gln Gln Gln Gln Met Arg Leu Gln Gln
            260                 265                 270

Leu Gln Met Glu Lys Glu Arg Leu Arg Leu Lys Gln Gln Glu Leu Leu
        275                 280                 285

Arg Gln Glu Leu Ala Leu Arg Ser Gln Leu Pro Thr Leu Glu Gln Asp
    290                 295                 300

Gly Gly Thr Gln Asn Pro Val Ser Ser Pro Gly Met Ser Gln Glu Leu
305                 310                 315                 320

Arg Thr Met Thr Thr Asn Ser Ser Asp Pro Phe Leu Asn Ser Gly Thr
                325                 330                 335

Tyr His Ser Arg Asp Glu Ser Thr Asp Ser Gly Leu Ser Met Ser Ser
            340                 345                 350

Tyr Ser Val Pro Arg Thr Pro Asp Asp Phe Leu Asn Ser Val Asp Glu
        355                 360                 365

Met Asp Thr Gly Asp Thr Ile Asn Gln Ser Thr Leu Pro Ser Gln Gln

370                 375                 380

Asn Arg Phe Pro Asp Tyr Leu Glu Ala Ile Pro Gly Thr Asn Val Asp
385                 390                 395                 400

Leu Gly Thr Leu Glu Gly Asp Gly Met Asn Ile Glu Gly Glu Glu Leu
                405                 410                 415

Met Pro Ser Leu Gln Glu Ala Leu Ser Ser Asp Ile Leu Asn Asp Met
            420                 425                 430

Glu Ser Val Leu Ala Ala Thr Lys Leu Asp Lys Glu Ser Phe Leu Thr
        435                 440                 445

Trp Leu
    450

<210>  16
<211>  454
<212>  PRT
<213>  Homo sapiens

<220>
<221>  MISC_FEATURE
<223>  Human YAP1-1 beta

<400>  16
   Met Asp Pro Gly Gln Gln Pro Pro Pro Gln Pro Ala Pro Gln Gly Gln
   1               5                   10                  15
   Gly Gln Pro Pro Ser Gln Pro Pro Gln Gly Gln Gly Pro Pro Ser Gly
               20                  25                  30
   Pro Gly Gln Pro Ala Pro Ala Ala Thr Gln Ala Ala Pro Gln Ala Pro
           35                  40                  45
   Pro Ala Gly His Gln Ile Val His Val Arg Gly Asp Ser Glu Thr Asp
       50                  55                  60
   Leu Glu Ala Leu Phe Asn Ala Val Met Asn Pro Lys Thr Ala Asn Val
   65                  70                  75                  80
   Pro Gln Thr Val Pro Met Arg Leu Arg Lys Leu Pro Asp Ser Phe Phe
                   85                  90                  95
   Lys Pro Pro Glu Pro Lys Ser His Ser Arg Gln Ala Ser Thr Asp Ala
               100                 105                 110
   Gly Thr Ala Gly Ala Leu Thr Pro Gln His Val Arg Ala His Ser Ser
           115                 120                 125
   Pro Ala Ser Leu Gln Leu Gly Ala Val Ser Pro Gly Thr Leu Thr Pro
       130                 135                 140
   Thr Gly Val Val Ser Gly Pro Ala Ala Thr Pro Thr Ala Gln His Leu
   145                 150                 155                 160
   Arg Gln Ser Ser Phe Glu Ile Pro Asp Asp Val Pro Leu Pro Ala Gly
                   165                 170                 175
   Trp Glu Met Ala Lys Thr Ser Ser Gly Gln Arg Tyr Phe Leu Asn His
               180                 185                 190
   Ile Asp Gln Thr Thr Thr Trp Gln Asp Pro Arg Lys Ala Met Leu Ser
           195                 200                 205
   Gln Met Asn Val Thr Ala Pro Thr Ser Pro Pro Val Gln Gln Asn Met
       210                 215                 220
   Met Asn Ser Ala Ser Ala Met Asn Gln Arg Ile Ser Gln Ser Ala Pro
   225                 230                 235                 240
   Val Lys Gln Pro Pro Pro Leu Ala Pro Gln Ser Pro Gln Gly Gly Val
                   245                 250                 255
   Met Gly Gly Ser Asn Ser Asn Gln Gln Gln Gln Met Arg Leu Gln Gln
               260                 265                 270
   Leu Gln Met Glu Lys Glu Arg Leu Arg Leu Lys Gln Gln Glu Leu Leu
           275                 280                 285
   Arg Gln Val Arg Pro Gln Glu Leu Ala Leu Arg Ser Gln Leu Pro Thr
       290                 295                 300
   Leu Glu Gln Asp Gly Gly Thr Gln Asn Pro Val Ser Ser Pro Gly Met
   305                 310                 315                 320
   Ser Gln Glu Leu Arg Thr Met Thr Thr Asn Ser Ser Asp Pro Phe Leu
                   325                 330                 335
   Asn Ser Gly Thr Tyr His Ser Arg Asp Glu Ser Thr Asp Ser Gly Leu
               340                 345                 350
   Ser Met Ser Ser Tyr Ser Val Pro Arg Thr Pro Asp Asp Phe Leu Asn
           355                 360                 365
   Ser Val Asp Glu Met Asp Thr Gly Asp Thr Ile Asn Gln Ser Thr Leu
       370                 375                 380
   Pro Ser Gln Gln Asn Arg Phe Pro Asp Tyr Leu Glu Ala Ile Pro Gly
   385                 390                 395                 400
   Thr Asn Val Asp Leu Gly Thr Leu Glu Gly Asp Gly Met Asn Ile Glu
                   405                 410                 415
   Gly Glu Glu Leu Met Pro Ser Leu Gln Glu Ala Leu Ser Ser Asp Ile
               420                 425                 430
   Leu Asn Asp Met Glu Ser Val Leu Ala Ala Thr Lys Leu Asp Lys Glu
           435                 440                 445
   Ser Phe Leu Thr Trp Leu
       450

<210>  17
<211>  23
<212>  DNA
<213>  Artificial

<220>
<223>  DNA encoding single chain guide RNA

<400>  17
ggagatctct gaagtgaaga tgg                                              23

<210>  18
<211>  163
<212>  DNA
<213>  Artificial

<220>
<223>  Single chain donor DNA for mutation

<400>  18
tggttgtcct gcatacttta attatgatgt aatacaggtt ctgggttgac aaatatcaag      60

acggaggaga tctctgaagt gaatctggat gcagaattcc gacatgactc aggatatgaa     120

gttcatcatc aaaaattggt acgtaaaata atttacctct ttc                       163

<210>  19
<211>  25
<212>  DNA
<213>  Artificial

<220>
<223>  Forward primer for PCR

<400>  19
gcatgtattt aaaggcagca gaagc                                            25

<210>  20
<211>  34
<212>  DNA
<213>  Artificial

<220>
<223>  Reverse primer for PCR

<400>  20
caatgcttgc ctataggatt accatgaaaa catg                                    34